# EUROPEAN PATENT APPLICATION

(11) **EP 2 048 140 A1**
(43) Date of publication of application: **15.04.2009**
(21) Application number: 08016385.0
(22) Date of filing: 17.07.2002
(51) Int. Cl.: C07D 321/00, C07F 9/655, C07B 59/00

(54) **Deuterated chemiluminescent 1,2-dioxetanes**

(30) Priority: 17.07.2001 US 306041 P
(62) Divisional of application: 02803264.7
(71) Applicant: Giri, Brij P., Sterling Heights, MI 48310 (US); Dagli, Dinesh, Troy, MI 48084 (US)
(72) Inventor: Giri, Brij P., Sterling Heights, MI 48310 (US); Dagli, Dinesh, Troy, MI 48084 (US)
(74) Representative: Burrows, Anthony Gregory

(57) **Abstract**

Deuterium-based i.e. isotopic hydrogen chemiluminescent 1,2-dioxetanes derived from the photo-oxidation of new novel alkenes which are synthesized by the coupling reaction of (a) a saturated or unsaturated cyclic, polycylic, normal or branched chain alkyl, cycloalkyl and spiro-fused and (b) substituted aromatic esters or ketones wherein (a) or (b) or both at least have a deuterium atom or a deuterium atom-containing group. These deuterium-based 1,2-dioxetanes may also have electron donating or withdrawing groups at the four-membered peroxide ring. Thus, the added electronic charge and the isotopic hydrogen or isotopic hydrogen-containing group hereof affects the light producing efficiency of 1,2-dioxetanes.

## Description

### Cross Reference to Related Application

This application is a completion application of copending U.S. provisional application Serial No. 60/306,041, filed July 17, 2001, the disclosure of which is hereby incorporated by reference.

### BACKGROUND OF THE INVENTION

### 1. FIELD OF THE INVENTION

The present invention relates to chemiluminescent compounds. More particularly, the present invention concerns stable; triggerable chemiluminescent 1, 2-dioxetanes and methods of preparation therefore. Even more particularly, the present invention concerns new deuterium-based chemiluminescent 1,2-dioxetane compounds derived from the photo-oxidation of novel new alkenes prepared by the coupling of (a) a saturated or unsaturated cyclic, polycyclic, normal or branched chain alkyl, cycloalkyl and spiro-fused ketone with or without deuterium atom or deuterium containing group and (b) substituted aromatic esters or ketones with or without deuterium atom or deuterium atom-containing group.

### 2. PRIOR ART

In copending U. S. Patent Appplication, Serial Nos. 09/883,586, the disclosure of which is incorporated by reference, there is provided a detailed history of the evolution of chemiluminescent compounds and their uses, which for the sake of brevity need not to be repeated herein.

However, for purposes of the ensuing description of the present invention, it should be noted that the enzyme cleavable 1, 2-dioxetanes of formulae (1), (2), (3), (4) and (5) shown below and disclosed in the copending application have been commercialized and used in immunoassays, Southern blotting, Northern blotting, Western blotting, plaque/colony lifts and DNA sequencing.

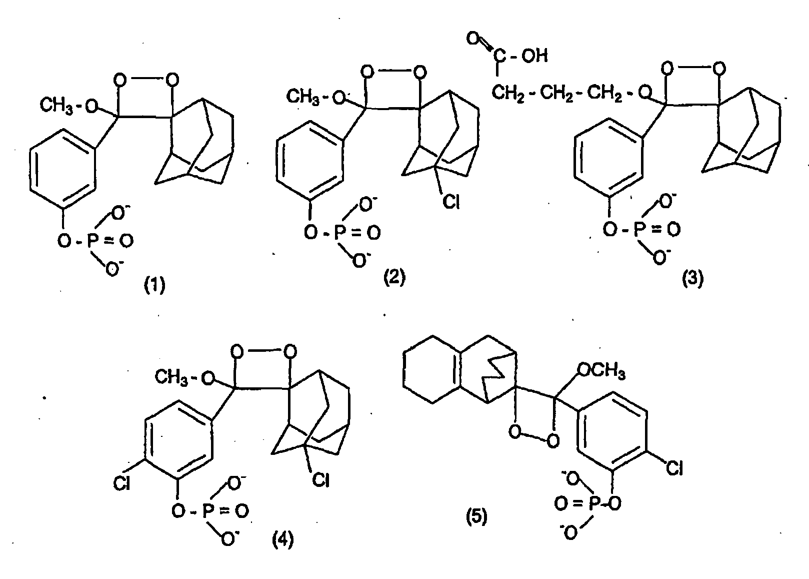

The 1, 2-dioxetanes of formulae (2) and (4) with chlorosubstitution in the adamantane ring or in both the adamantane ring and the phenyl ring demonstrate better results in DNA sequencing when compared to the dioxetane of formula (1). Dioxetane (3) is more soluble in an aqueous system than those of formulae (1), (2) and (4) when a CH₃ group is replaced with a CH₂CH₂CH₂COOH and dioxetane (5) with chlorosubstitution in the benzene ring and unsaturation in the adamantane ring and, specifcally tricyclo [7.3.1.0^{2,7}] tridec-2, 7-ene-1 3-one has demostrated greater superiority in sensitivity on membranes as well as in other applications.

While these and the other prior art compounds provide stable enzyme cleavable 1, 2-dioxetanes, it has been observed that in an aqueous buffer the luminescence of these molecules is particularily poor, especially when trace amounts of biological materials are sought to be detected. Thus more powerful dioxetanes are needed i.e. dioxetanes having higher levels of chemiluminescence in an aqueous buffer.

### SUMMARY OF THE INVENTION

The present invention provides novel deuterated substituted 1, 2-dioxetanes derived from substituted alkenes which are synthesized by the coupling of (a) a saturated or unsaturated cyclic, polycyclic, normal or branched chain alkyl, cycloalkyl or spiro-fused and (b) substituted aromatic esters or ketones wherein at least one or both of (a) and (b) contain a deuterium atom or a deuterium atom-containing group. Additionally, these new deuterated substituted 1, 2-dioxetanes hereof may have electron donating or withdrawing groups with at least one of the hydrogen atoms replaced by a deuterium atom or a deuterium atom-containing group.

The 1, 2-dioxetanes hereof generally correspond to the formula: 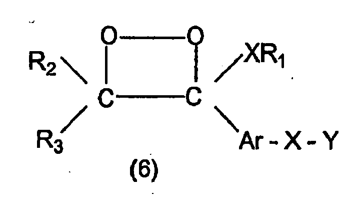 wherein at least one of R₁, R₂ or R₃ and Ar comprises a deuterium atom or deuterium atom-containing group, X is sulfur or oxygen and,
wherein:
(a) when R₂ and R₃ form either (I) 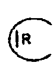 which is a cyclic, polycyclic or spiro-fused ring containing at least one carbon-carbon double bond or cabon-carbon triple bond in the ring or side chain, with or without heteroatoms, or (II) 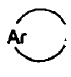 which is a cyclic, polycyclic or a spiro-fused ring containing a substituted or unsubstituted fused aromatic ring or a substituted or unsubstituted aromatic ring attached by linker arms, or (III) 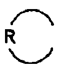 which is either a cyclic, substituted or unsubstituted polycyclic alkyl group which is spiro-fused to the dioxetane ring or (IV) R₂ and R₃ are each substituted or unsubstituted branched alkyl groups or cycloalkyl groups having 3 to 8 carbon atoms and being substituted in the ring or side chain, Ar is an aryl group which may be phenyl, substituted phenyl, naphthyl, substituted naphthyl, anthryl, substituted anthryl with or without a fluorescent group; Y is either hydrogen, alkyl, acetate, t-butyldimethylsilyl, an enzyme cleavable group, or an antibody cleavable group; and R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, inker-avidin, linker- protein, linker- carbohydrate or linker- lipid, or
(b) when R₂ and R₃ contain an isotopic hydrogen and either form (I) 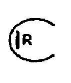 which is a cyclic, polycyclic or a spiro-fused ring comprised of at least one carbon-carbon double bond or cabon-carbon triple bond in the ring or side chain with or without heteroatoms, (II) 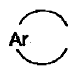 which is a cyclic, polycyclic or spiro-fused ring which is a substituted or unsubstituted fused aromatic ring or substituted or unsubstitued aromatic ring attached by linker arms, or (III) 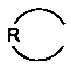 which is either a substituted or unsubstituted cyclic, or polycyclic alkyl group which is spiro- fused to the dioxetane ring or (IV) R₂ and R₃ each contain 3 to 8 carbon atoms and are substituted or substituted branched alkyl or cycloalkyl groups having substitution in the ring or side chain, Ar, X and Y are as above; and R₁ is an organic group which is either cyclic, linear or branched, halogenated or non-halogenated alkyl, aryl, aralkyl, alkaryl, heteroalkyl, heteroaryl, cycloalkyl, , cycloheteroalkyl, alkyletheralkyl, alkletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker- protein, linker-carbohydrate or linker- lipid, or
(c) when R₂ and R₃ form either (I) 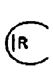 which is a cyclic, polycyclic or spiro-fused ring containing at least one carbon-carbon double bond or cabon-carbon triple bond in the ring or side chain, with or without heteroatoms, (II) 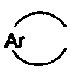 which is a cyclic, polycyclic or a spiro-fused ring containing a substituted or unsubstituted fused aromatic ring or a substituted or unsubstituted aromatic ring attached by linker arms, or (III) 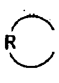 which is either a cyclic, a substituted or unsubstituted polycyclic alkyl group which is spiro-fused to the dioxetane ring or (IV) R₂ and R₃ are organic group each having 3 to 8 carbon atom and may be branched alkyl or cycloalkyl groups having substitution in the ring or side chain, Ar is an aryl group containing an isotopic hydrogen or isotopic hydrogen containing group, which may be phenyl, substituted phenyl, naphthyl, substituted naphthyl, anthryl, substituted anthryl or any other aromatic or nonaromatic fluorescent or nonfluorescent group; X and Y are as defined above, and R₁ is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnkrile, haloalkylamine, haloalkylacid or inorganic salts thereof, linker-flourescent molecule, linker-antibody, linker antigen, linker-blotin, linker-avidin, linker- protein, linker- carbohydrate or linker- lipid.

These new 1, 2-dioxetanes are triggered by the same activating agents described in the above referenced co-pending application and in the other cited prior art, the disclosures of which are incorporated by reference and which agents are well known in the prior art.

These new 1, 2-dioxetanes are prepared from new alkenes having isotopic hydrogen or an isotopic hydrogen-containing group. Preferably, these alkenes are prepared by the reaction of (a) adamantan-2-one or other spiro-fused ketone having a carbon to carbon double or triple bond in the ring or side chain with (b) a substituted aromatic ester or other ketone, wherein at least one or both of the spiro-fused ketone or the aromatic ester or other ketone has an isotopic hydrogen or an isotopic hydrogen-containing group, using titanium trichloride or tetrachloride and a reducing agent such as an active metal or lithium aluminium hydride in tetrahydrofuran (THF) or other solvent of choice. This reaction is an intermolecular coupling of a ketone and an ester or other ketone to form a vinyl ether using a modified McMurray procedure, as described in J. Am. Chem. Soc., 105, 1660-61, (1983) for intramolecular coupling of a ketone and ester. Ordinarily, the reactants are present in at least stoichiometric quantities. However, excess amounts of the ester or ketone can be used. The temperatures at which the reactions as described above are those disclosed in the art.

As is seen from the above and as noted, in order to prepare the alkenes hereof at least one of either the spiro-fused ketone or the aromatic ester or other ketone must have an isoptopic hydrogen or isotopic hydrogen-containing group. If the spiro-fused ketone does not have isotopic hydrogen, preferably, it is an unsaturated compound with either a carbon-to-carbon double or triple bond.

Photo-oxygenation of the resulting vinyl ethers by well-known conventional techniques affords 1, 2-dioxetanes that are easily handled compounds with the desired stability.

The chemiluminescent 1, 2-dioxetanes hereof, as noted above, can, preferably, be conveniently triggered at room temperature by removing the protecting group with a fluoride ion, a base or an enzyme to generate an unstable aryl oxide 1, 2-dioxetane intermediate which cleaves to the starting materials and yields intense blue or other colored luminescence light.

### BRIEF DESCRIPTION OF THE DRAWINGS

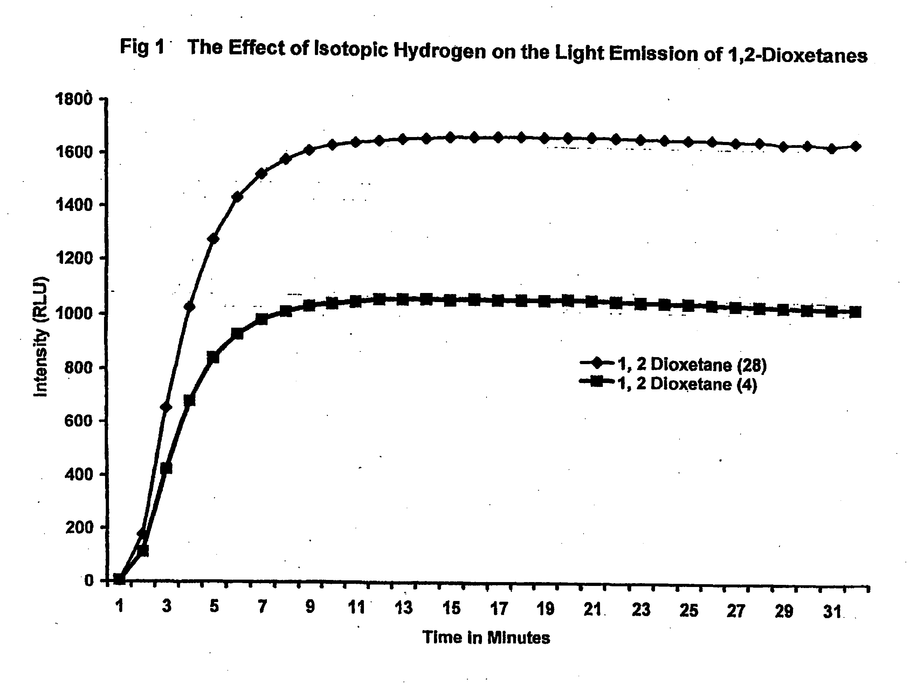

FIG. 1 is a graph comparing the effect of isotopic hydrogens on the light emission of a present 1, 2-dioxetane (28)when compared to the 1, 2-dioxetane of formula (4), when triggered by alkaline phosphatase enzyme in Tris buffer.

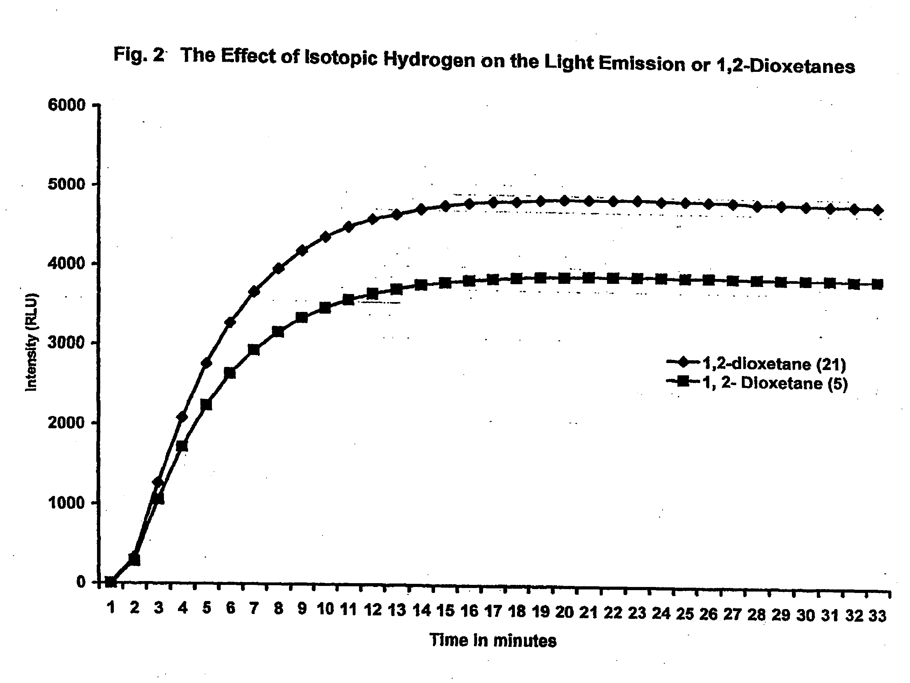

FIG. 2 is a graph comparing the effect of isotopic hydrogens on the light emission of a present 1, 2-dioxetane (21) when compared to the 1, 2-dioxetane of formula (5), when triggered by alkaline phosphatase enzyme in Tris buffer.

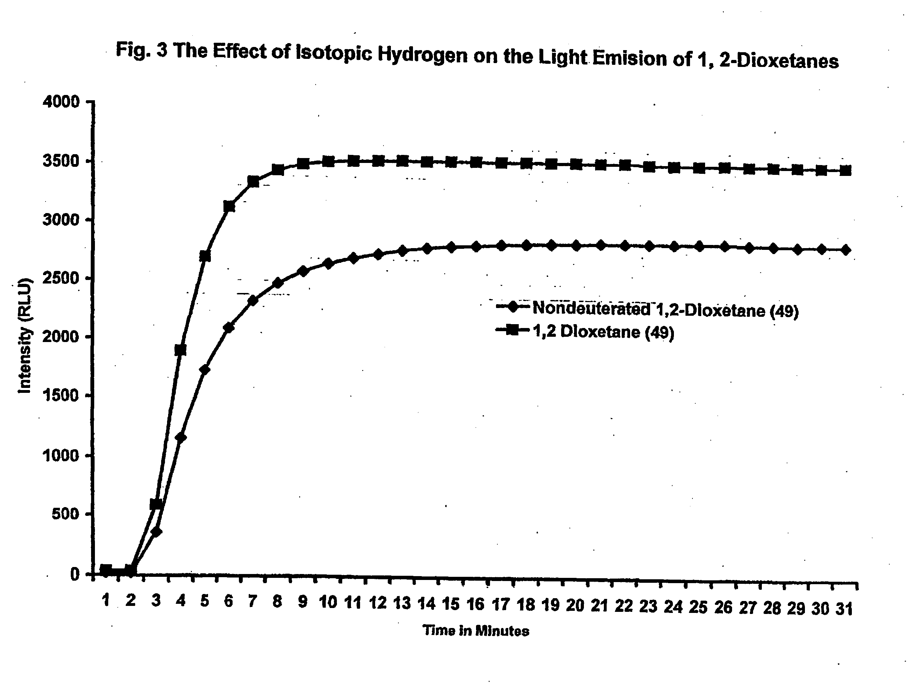

FIG. 3 is a graph comparing the effect of isotopic hydrogens on the light emission of a present 1, 2-dioxetane (49) when compared to the give [(4-Methoxy-4-(3-phosphoryloxy-4-chlorophenyl)] spiro [1,2-dioxetane-3,2'-adamantane], disodium salt, when triggered by alkaline phosphatase enzyme in Tris buffer.

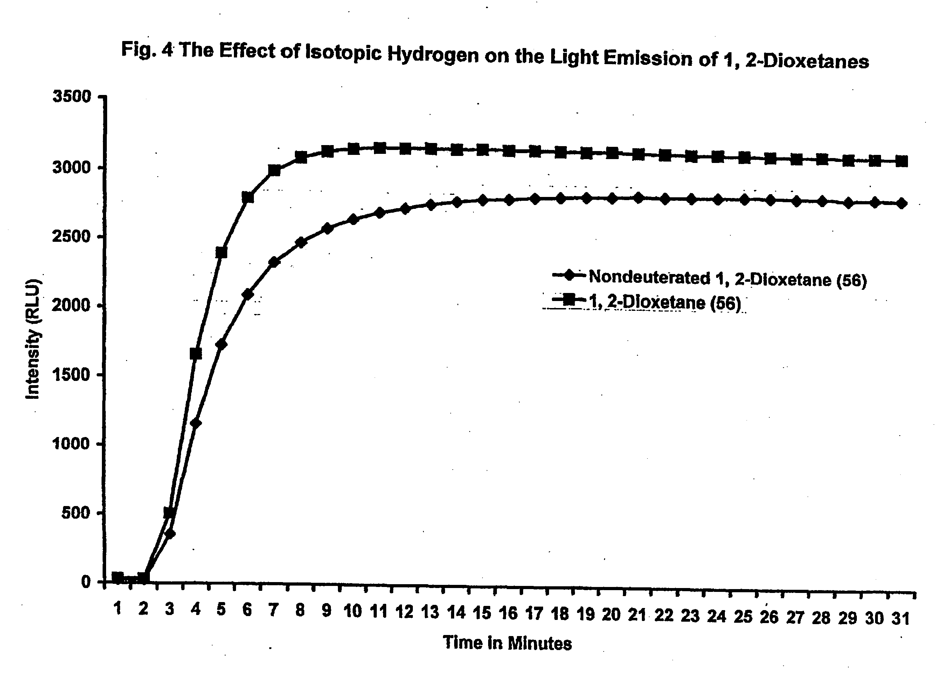

FIG. 4 is a graph comparing the effect of isotopic hydrogens on the light emission of a present 1, 2-dioxetane (56) when compared to the give [4-Methoxy-4-(3-phosphoryloxy-4-chlorophenyl)] spiro [1,2-dioxetane-3,2'-admantane], disodium salt, when triggered by alkaline phosphatase enzyme in Tris buffer.

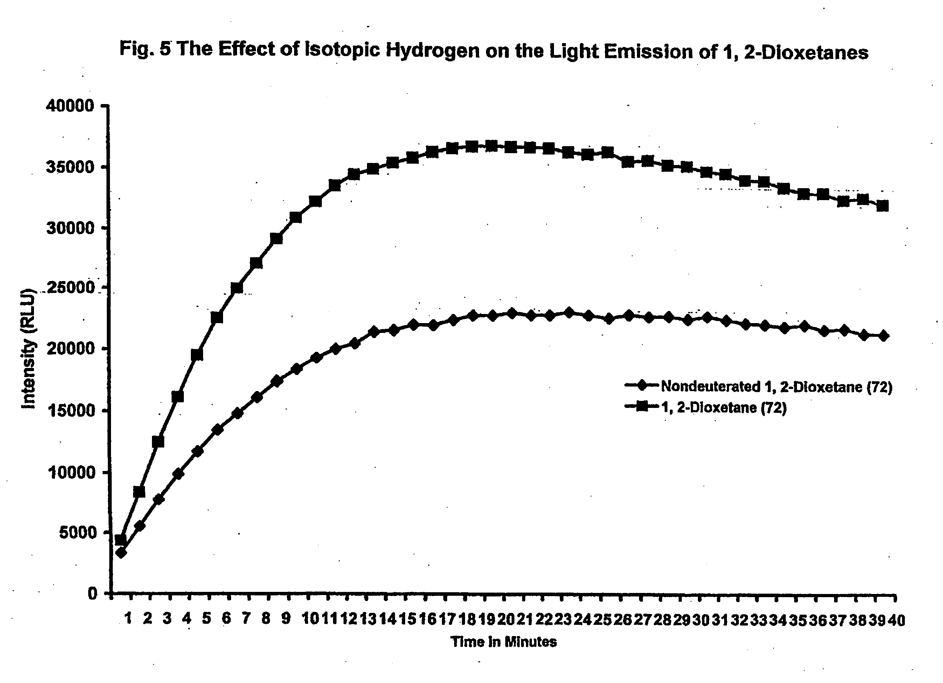

FIG. 5 is a graph comparing the effect of isotopic hydrogens on the light emission of a present 1, 2-dioxetane (72) when compared to the give [4-Methoxy-4-(3-phosphoryloxy-5-methoxyphenyl)] spiro[1,2-dioxetane-3,2'-admantane], disodium salt, when triggered by alkaline phosphatase enzyme in Tris buffer.

### DESCRIPTION OF PREFERRED EMBODIMENTS

As stated above chemiluminescent enzyme cleavable substrates based on 1, 2- dioxetane are well known in the literature for biological assays such as immunoassays, blotting and DNA probes. Use of these high-energy compounds in biological systems requires I, 2-dioxetanes which are thermally stable at the temperature of the enzymatic reaction and which do not undergo rapid spontaneous decompostion in an aqueous buffer. The 1, 2- dioxetanes hereof meet these requirements.

The present 1, 2-dioxetanes can be modified as substrates for various enzymes including aryl esterase, β-galactosidase, alkaline phosphatase and others.

At the outset, it should be noted that the terms "deuterium atom" and "isotopic hydrogen" are used interchangeably throughout the specification and in the appended claims.

In accordance herewith and as noted above, the present invention provides new 1, 2-dioxetanes. The new 1, 2-dioxetanes hereof correspond to the formula: 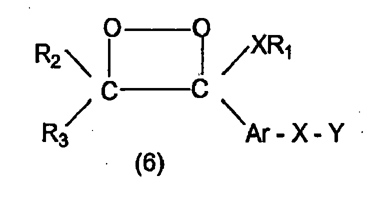 wherein at least one of R₁, R₂ or R₃ and Ar comprises a deuterium atom or deuterium atom containing group, X is sulfur or oxygen and,
wherein:
(a) when R₂ and R₃ form either (I) 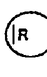 which is a cyclic, polycyclic or spiro-fused ring containing at least one carbon-carbon double bond or cabon-carbon triple bond in the ring or side chain, with or without heteroatoms, or (II) 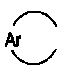 which is a cyclic, polycyclic or a spiro-fused ring containing a substituted or unsubstituted fused aromatic ring or a substituted or unsubstituted aromatic ring attached by linker arms, or (III) 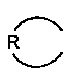 which is either a cyclic, substituted or unsubstituted polycyclic alkyl group which is spiro-fused to the dioxetane ring or (IV) R₂ and R₃ are each substituted or unsubstituted branched alkyl groups or cycloalkyl groups having 3 to 8 carbon atoms and being substituted in the ring or side chain, Ar is an aryl group which may be phenyl, substituted phenyl, naphthyl, substituted naphthyl, anthryl, substituted anthryl with or without a fluorescent group; Y is either hydrogen, alkyl, acetate, t-butyldimethylsilyl, an enzyme, an enzyme cleavable group, or an antibody cleavable group; and R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, inker-avidin, linker- protein, linker- carbohydrate or linker- lipid, or
(b) when R₂ and R₃ contain an isotopic hydrogen and either form (I) 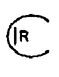 which is cyclic, polycyclic or a spiro-fused ring comprised of at least one carbon-carbon double bond or cabon-carbon triple bond in the ring or side chain with or without heteroatoms, (II) 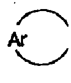 which is a cyclic, polycyclic or spiro-fused ring which is a substituted or unsubstituted fused aromatic ring or substituted or unsubstituted aromatic ring attached by linker arms, or (III) 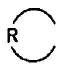 which is either a substituted or unsubstituted cyclic, or polycyclic alkyl group which is spiro- fused to the dioxetane ring or (IV) R₂ and R₃ each contain 3 to 8 carbon atoms and are substituted or unsubstituted branched alkyl or cycloalkyl groups having substitution in the ring or side chain, Ar, X and Y are as above; and R₁ is an organic group which is either cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker protein, linker- carbohydrate or linker- lipid, or
(c) when R₂ and R₃ form either (I) 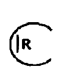 which is a cyclic, polycyclic or spiro-fused ring containing at least one carbon-carbon double bond or cabon-carbon triple bond in the ring or side chain, with or without heteroatoms, (II) 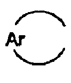 which is a cyclic, polycyclic or a spiro-fused ring containing a substituted or unsubstituted fused aromatic ring or a substituted or unsubstituted aromatic ring attached by linker arms, or (III) 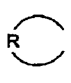 which is either a cyclic, a substituted or unsubstituted polycyclic alkyl group which is spiro-fused to the dioxetane ring or (IV) R₂ and R₃ are each 3 to 8 carbon atoms are substituted or unsubstituted branched alkyl or cycloalkyl groups having substitution in the ring or side chain, Ar is an aryl group containing an isotopic hydrogen or isotopic hydrogen containing group, which may be phenyl, substituted phenyl, naphthyl, substituted naphthyl, anthryl, substituted anthryl or any other aromatic or nonaromatic fluorescent or nonfluorescent group; X and Y are as defined above, and R₁ is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, tinker-antigen, linker-biotin, linker-avidin, linker- protein, linker- carbohydrate or linker- lipid.

The 1, 2-dioxetanes hereof are prepared from alkenes having an isotopic hydrogen or isotopic hydrogen atom-containing group thereof. These alkenes are synthesized by the coupling of (a) a saturated or unsaturated cyclic, polycyclic, normal or branched chain alkyl, cycloalkyl and spiro-fused ketone with or without deuterium atom or deuterium containing group and (b) substituted aromatic esters or ketones with or without deuterium atom or deuterium containing group. Generally, the reaction proceeds, using titanium trichloride or tetrachloride and a reducing agent such as an active metal or lithium aluminium hydride in tetrahydrofuran (THF) or other solvent of choice. This reaction is an intermolecular coupling of a ketone and an ester or ketone to form vinyl ether using a modified McMurray procedure. Ordinarily, the reactants are present in at least stoichiometric quantities. However, excess amounts of the ester or ketone can be used. The temperatures at which the reactions as described above are those disclosed in the art.

The new alkenes used to prepare the present 1, 2-dioxetanes correspond to the general formula: 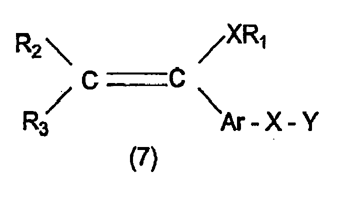 Wherein R₁, R₂, R₃, Y, X and Ar are as described above,

These alkenes are prepared by the coupling of the above- described ketones and esters or ketones to form a vinyl ether. The coupling reaction is carried out in the presence of suitable solvents and active metals as described in the prior art denoted above, the disclosures of which are hereby incorporated by reference.

Generally, the intermolecular coupling reaction between the spiro-fused ketone or ketones and the ester or other ketone is carried out at a temperature ranging from about 25°C to about 85°C and, preferably, from about 45°C to about 65°C. A stoichiometric excess of either the ester or ketone may be used.

After the alkene is obtained it is then, photooxidized to form the stable, triggerable 1, 2- dioxetanes hereof. These dioxetanes can, then, be de-stablized or triggered by reaction with a base, an acid, enzyme, inorganic or organic catalyst and / or electron donor source in the presence or absence of a fluorescent compound.

These present 1, 2-dioxetanes when reacted with an activating reagent or agent which removes the Y moiety, form an unstable intermediate 1, 2-dioxetane of the formula:

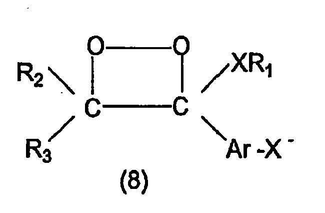

When X is oxygen, the aryloxy 1, 2-dioxetane intermediate, then, spontaneously decomposes to produce light and compounds of the formulae: 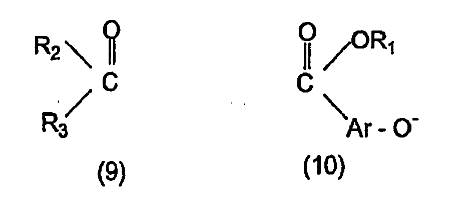 where compound (9)) a saturated or unsaturated cyclic, polycyclic, normal or branched chain alkyl, cycloalkyl and spiro-fused ketone with or without deuterium atom or deuterium containing group and (10) is substituted aromatic esters or ketones with or without deuterium atom or deuterium atom-containing group

In practicing the present invention, representive compounds corresponding to formula (9) include for example an adamantan-2-one, substituted adamantan-2-one, adamantan-2-one-4, 5-ene, substituted adamantan-2-one-4, 5-ene, 2-hydroxytricyclo [7.3. 1.0^{2,7}] tridecan-1 3-one, substituted 2-hydroxytricyclo [7.3.1.0^{2,7}] tridecan-1, 3-one, tricyclo(7.3.1.0^{2,7}] tridec-2,7-ene -13-one, substituted tricyclo [7.3.1.0^{2,7}] tridec-2,7-ene -13-one, bicyclo[3.3. 1] nonan-9-one, substituted bicyclo[3.3.1]nonan-9-one, benzonorbonen-7-one, substitutrd benzonorbomen-7-one, 2,4-dimethyl-3-propanone, substituted 2,4-dimethyl-3-propanone, dicyclopropyl ketone, substituted dicyclopropyl ketone, dicyclohexylketone, substituted dicyclohexyl ketone and the like, as well as mixtures thereof,

Compound (10) is, preferably, selected from the group consisting methyl (D₃) -3-hydroxybenzoate, substituted consisting methyl (D₃) -3-hydroxybenzoate, consisting methyl (D₂) -3-hydroxybenzoate, consisting methyl (D₁) -3-hydroxybenzoate, 2,2,2-trifluoroethyl (D₂)-3-hydroxybenzoate, substituted 2,2,2-trifluoroethyl (D₂)-3- hydroxybenzoate, 2-phenoxyethyl 3-hydroxybenzoate, substituted 2-phenoxyethyl 3-hydroxybenzoate, Methyl 3-hydroxy-5-methoxy (D₃) benzoate, Methyl 3-hydroxy-5-methoxy (D₃)-4-chlorobenzoate, Methyl 3-hydroxy-5-methoxy (D₃)-4 bromo -benzoate,Methyl 3-hydroxy-5-methoxy (D₃)-4-fluorobenzoate Methyl 3-hydroxy-5-methoxy (D₃)-4-cyanobenzoate and the like, as well as mixtures thereof,

When the ketone of formula (9) is selected from the group consisting of adamantan-2-one, substituted adamantan-2-one, adamantan-2-one-4, 5-ene, substituted adamantan-2-one-4,5-ene, tricyclo [7.3.1.0^{2,7}] tridec-2, 7-ene -13-one, substituted tricyclo [7.3.1.0^{2,7}] tridec-2,7-ene -13-one, compound (10) is alkyl (D) or aryl (D) 3-hydroxybenzoate, substituted alkyl (D), aryl (D)-3-hydroxybenzoate and the like, as well as mixtures thereof. When the ketone of formula (9) is selected from the group consisting of adamantan-2-one(D), substituted adamantan-2-one(D), adamantan-2-one-4,5-ene(D), substituted adamantan-2-one-4,5-ene(D), tricyclo [7.3.1.0 ^{2,7}] tridec-2,7-ene -13-one(D), substituted tricyclo [7.3.1.0^{2,7}] tridec-2,7-ene - 13-one(D), compound (10) is alkyl or aryl 3-hydroxybenzoate, substituted alkyl, aryl-3-hydroxybenzoate and the like, as well as mixtures thereof. When the ketone of formula (9) is selected from the group consisting of adamantan-2-one(D), substituted adamantan-2-one(D), adamantan-2-one-4,5-ene(D), substituted adamantan-2-one-4,5-ene(D), tricyclo [7.3.1.0^{2,7}] tridec-2,7-ene -13-one(D), substituted tricyclo [7.3.1.0^{2,7}] tridec-2,7-ene -13-one(D), compound (10) is alkyl(D) or aryl(D) 3-hydroxybenzoate, substituted alkyl(D), aryl(D)-3-hydroxybenzoate and the like, as well as mixtures thereof.

Preferably, compound (9) is adamantane-2-one, 5-chloroadamantan-2-One, adamantan-2-one-4, 5-ene, 5-methoxy (D₃) adamantan-2-one, 5-chloromethoxy adamantan-2-one, 5-methoxy adamantan-2-one, 5-trifluoroethoxyadamantan-2-one, tricyclo (7.3.1.0^{2,7}] tridec-2, 7-ene -13-one and mixtures thereof, and compound (10) is methyl (D₃)-3-hydroxybenzoate, methyl (D₃)-3-hydroxy-4-chlorobenzoate,methyl 3-hydroxy-4-chlorobenzoate, methyl 3-hydroxybenzoate, Methyl 3-hydroxy-5-methoxy (D₃) benzoate, Methyl 3-hydroxy-5-methoxybenzoate, and mixtures thereof.

Most preferably, the spiro-fused ketone is adamantane-2-one, 5-chloroadamantan-2-one or tricyclo [7.3.1.0^{2,7}] tridec-2, 7-ene-13-one, adamantan-2-one-4, 5-ene, 5-methoxy (D₃) adamantan-2-one, 5-methoxyadamantan-2-one and the coupling compound is methyl (D₃)-3-hydroxybenzoate, methyl (D₃) 3-hydroxy-4-chlorobenzoate, methyl 3-hydroxy-4-chlorobenzoate, methyl 3-hydroxy-5-methoxy (D₃) benzoate.

It should be noted that in synthesizing the coupling ester, it is necessary, as is known to the skilled in the art, that initially an intermediate having a pendent hydroxyl group will be formed which is, then, further react with t-butyldimethylsilyl chloride or other protecting group for protect the hydroxyl group to reaction with the coupling ketone.

In use, the 1, 2- dioxetanes are, preferably, deployed as an alkaline salt. In preparing the 1, 2- dioxetanes salt, after photo-oxidation, the co-produced 1, 2-dioxetane is then treated with a base such as sodium hydroxide or sodium carbonate or potassium carbonate or the like.

For a more complete understanding of the present invention, reference is made to the following non-limiting examples. In the examples, all parts and percentages are by weight unless expressly stated to be otherwise.

In supporting the findings reported below, the structures of the resulting compounds were confirmed by Nuclear Magnetic Resonance (NMR). NMR spectra were recorded on a Brucker BZH 250 spectrometer in desired solvents using tetramethylsilane as an internal standard. Chemiluminescence kinetics were performed on a Monolight 1500 at room temperature. The purity of the materials was checked by TLC on silica gel plate. Melting points were measured in a MEL-TEMPII capillary melting point apparatus and are uncorrected. All the alkenes were dissolved in a suitable solvent and photooxidized by irradiation with 1000-W sodium lamp under bubbled oxygen at ice-water temperature in the presence of polystyrene-bound Rose Bengal as reported In the literature.

Chemiluminescence intensities were performed using a Monolight 1500 at room temperature. A buffer was prepared by dissolving one molar of tris (hydroxymethyl) aminomethane, 2.0 mM magnesiumchloride, 0.2mM zinc chloride in deionized water to form a 1 liter volumeteric aqeous buffer solution. The pH of the buffer was adjusted to pH 9.6 to pH 9.7 using concentrated hydrochloric acid. A solution contaning 0.4mM of the sample 1,2-dioxetane was prepared in the buffer. A 200µl sample was transferred into a tube, placed in the luminometer and background luminescence was recorded. Then 10µl of alkaline phosphatase diluted in tris buffer was added thereto. The luminescence was recorded at 60 second intervals to a maximum intensity. Figures 1, 2, 3, 4 and 5 show the results thereof.

### Example I

This example illustrates the preparation of [(4-Methoxy (D₃))-4-(3-phosphoryloxy-4-chlorophenyl)] spiro [1,2-dioxetane-3,13-tricyclo[7.3.1.0^{2,7}] tridec-2, 7-ene], disodium salt (21).
(a). Synthesis of methyl (D₃) 3-hydroxy-4-chlorobenzoate (12), (a starting intermediate ester).
   Into a 500mL round bottom flask equipped with magnetic stirrer and heating mantle was added 12 parts of 3-hydroxy-4-chlorobenzoic acid (11), 50mL of commerically avaliable deuterated methanol and 20 drops of concentrated sulfuric acid. The reaction mixture was heated under gentle reflux for 12 hours. The solvent was evaporated under reduced pressure and the recovered solid material was dissolved In 500 mL of ethyl acetate. The organic layer was washed with 250mL of water, 250mL of 5% aqueous solution of sodium bicarbonate and 250 mL of water. The ethyl acetate layer was dried over anhydrous sodium sulfate and the solvent was evaporated under reduced pressure. The oily material was treated with n-hexane to give a solid material which when filtered under reduced pressure and dried at room temperature, yield 10.5 parts. TLC on silica gel plate showed a single spot. The structure of the compound was confirmed on the basis of ¹H NMR. The material was prepared according to the following equation: 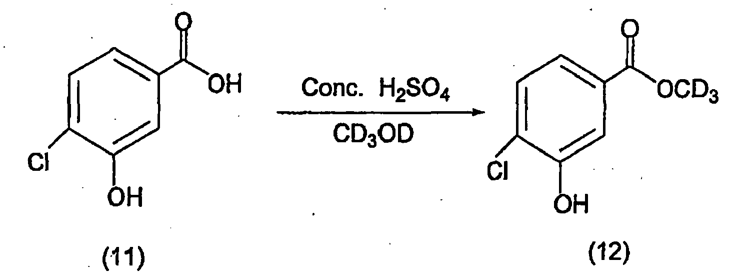
(b). Synthesis of methyl 3-tert-butyldimethylsiloxybenzoate (13), (a coupling ester).
   Into a 250 mL round bottom flask was added 50 mL of dry dimethyl formamide. Nine parts of the above-prepared hydroxy benzoate and seventeen parts of tert-butyldimethyl silyl chloride were added to the reaction flask with stirring. Ten parts of imidazole was added in portions and stirring was continued for 10 hours. The reaction mixture was extracted with (3 x 300 mL) of hexane and the hexane layer was washed twice with 250 mL of water. The organic layer was dried on anhydrous sodium sulfate and the solvent was evaporated under reduced pressure to give oil, yield 14.7 parts. This material was pure enough for the next step of the reaction. The material was prepared according to the following equation. 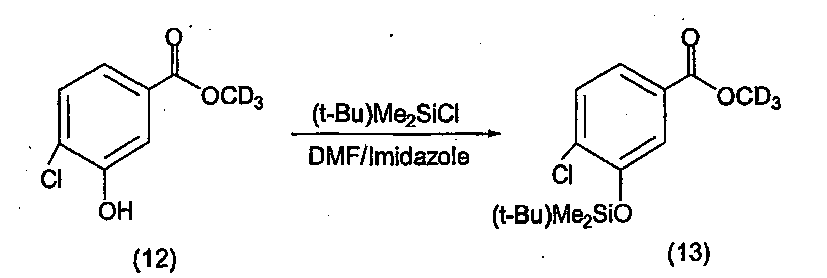
(c). Synthesis of 2-Hydroxytricyclo [7.3.1.0^{2,7}] tridecane-13-one (16), (a starting intermediate ketone).
   Into a 500 mL three-neck round bottom flask equipped with magnetic stirrer and oil bath was added 250 parts of cyclohexanone (14) under nitrogen. The oil bath temperature was maintained at 80-85°C with stirring. Sixty parts of absolute ethanol with 2.5 parts of potassium hydroxide was added in one portion. Sixteen parts of paraformaldehyde (15) was added in small portions over a period of 3 hours and the temperature was maintained for 15 hours with stirring. The reaction mixture was cooled to room temperature and refrigerated at 4°C for 15 hours. The inside surface of the flask and liquid was scratched with a glass rod. The solid started to separate and the flask was refrigerated for an additional 24 hours. The recovered solid was filtered under reduced pressure. The solid material was washed with 50mL of water and two times with 50mL of cold ether and dried, yield of 29 parts. The mother liquor was stored at -20° C for 15 hours. The solid material was filtered and washed with water and ether, yield 10.0 parts. The combined solid was washed with hexane. The material showed a single spot on TLC on a silica gel plate. The structure was confirmed on the basis of ¹H NMR. The reaction proceeded as follows: 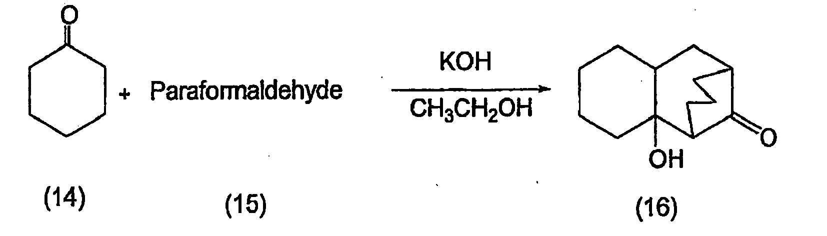
(d). Synthesis of tricyclo [7.3.1.0^{2,7}] tridec-2, 7-ene-1 3-one (17), (a coupling ketone).
   Into a 500mL round bottom flask was added 7.5 parts of the hydroxy ketone (16). The product was dissolved in 150mL of benzene by heating at-40°C. The reaction mixture was stirred at refluxing temperature for 12 hours in the presence of 0.5 parts of concentrated sulfuric acid. The reaction mixture was cooled to room temperature and the benzene solution was filtered. The solvent was evaporated under reduced pressure and the oily material dissolved in 150mL of methylene chloride. The organic layer was washed with water and dried on anhydrous sodium sulfate. The solvent was evaporated under reduced pressure and purified on silica gel column using 10% ethyl acetate/hexanes. The desired fractions were combined and the solvent was evaporated to give oil, yield 5.5 parts. ¹H NMR confirmed the following structure. The reaction proceeded as follows: 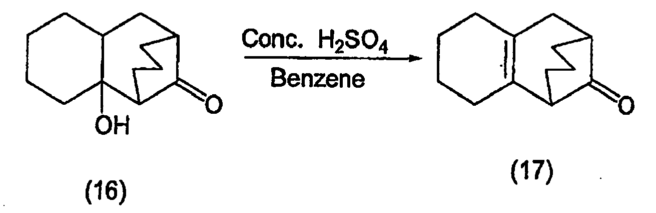
(e). Synthesis of (3-tert-butyldimethylsiloxyphenyl) methoxy (D₃) methylenetricyclo [7.3.1.0 ^{2,7}] tridec-2,7-ene (18), (a coupled intermediate alkene).
   Into a 500mL three-neck flask equipped with magnetic stirrer, pressure-equalizing addition funnel and nitrogen line was charged with 200mL of anhydrous THF. Twelve parts of titanium tetrachloride was added dropwise over a period of 30 minutes. The suspension was stirred for 20 minutes and 17 parts of zinc was added in small portions. The reaction mixture was heated under reflux for 2 hours and 30mL of triethylamine was added dropwise. After refluxing one hour, 4 parts of a solution of methyl (D₃) 3-tert-butyldimethylsiloxybenzoate (13) and 2 parts of ketone (17) in 35mL of dry THF was added over a period of 60 minutes and the reaction mixture was heated for one hour. The mixture on TLC silica gel plate showed the presence of the starting ester. An additional 1.5 parts of starting ketone (17) in 35mL of dry THF was added dropwise over 90 minutes and refluxed for two hours. The mixture was cooled to room temperature, diluted with 500mL of hexane and decanted. The residue was washed with 3 x 200mL of hexane. The combined hexane layer was filtered and evaporated under reduced pressure to give an oily material which was purified by chromatography on silica gel column using 2.5% ethyl acetate I hexane as an eluant. The fractions were checked by TLC on a silica gel plate and the desired fractions were combined. The solvent was evaporated under reduced pressure to give 4.75 parts of an oil substance. The structure was confirmed by ¹H NMR. The reaction proceeded as follows: 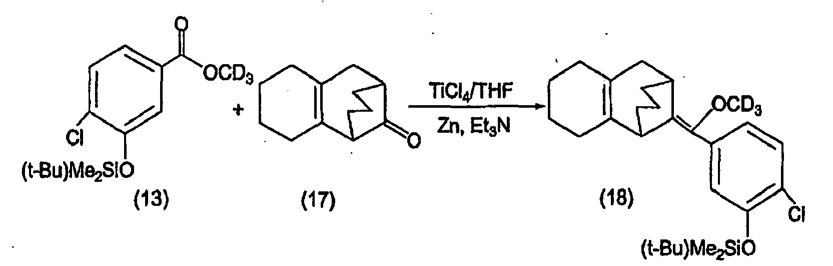
(f). Synthesis of (3-hydroxyphenyl) methoxy (D₃) methylene tricyclo [ 7.3.10^{2,7}] tridec-2,7-ene(19)(alkene).
   To a crude solution of 4 parts of (3-tert-Butyldimethylsiloxyphenyl) methoxy (D₃) methylene tricyclo [7.3.1.0^{2,7}] trideo-2,7-ene (19) in 150mL of THF was added 5.5 parts of 70% tetra-n-butylammonium fluoride in 50mL of THF over a period of 10 minutes and stirring was continued for two hours. TLC on silica gel plate showed the formation of new product. Solvent was evaporated under reduced pressure and the oily material was dissolved in 300 mL of methylene chloride and washed with 2 x 150mL of water. After drying over anhydrous sodium sulfate, the solvent was evaporated and the oily product was purified on silica gel column. TLC on silica gel plate of the fractions were checked and the combined organic solvent was evaporated, yield 2.60 parts. The structure of the product was confirmed on the basis of ¹H NMR. The reaction proceeded as follows: 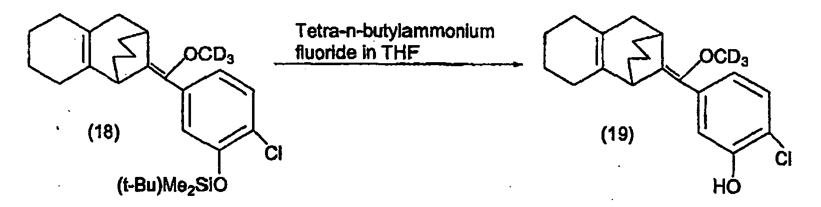
(g). Synthesis of (3-phosphoryloxyphenyl) methoxy (D₃) methylene tricyclo [7.3.1.0^{2,7}] tridec-2, 7-ene, disodium salt (20).
   Into a 250mL three-neck round bottom flask equippd with magenetic stirrer was added 15mL of dry THF under nitrogen and then 1mL of phosphorous oxychloride was added drop-wise (reaction flask was cooled in ice-water bath). A solution of 1 part of alkene (19) in 15mL of THF containing 0.325 parts of anhydrous pyridine was added to the reaction flask over a period of 30 minutes. The reaction mixture was stirred at room temperature for 3 hours. TLC on silica gel plate showed the formation of new product The solvent was evaporated to dryness and 10mL of THF was added to the reaction flask. A solution containing 0.55 parts of 3-hydroxypropionitrile and 0.65 parts of anhydrous pyridine in 7.5ml of dry THF was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column using 70% ethyl acetate I hexane containing 0.1 % triethylamine. Fractions were checke by TLC on silica-gel plate and the desired fractions were combined and evaporated under reduced pressure to give oil, yield 1.20 parts. The oily material was dissolved in 15mL of dry THF and a solution of 0.75 parts of sodium hydroxide in 5mL of water was added dropwise. Stirring was continued for two hours and the reaction mixture was diluted with 10mL of acetonitrile. The solid was filtered and washed with acetonitrile. The solid material was crystallized with a methanol and acetone mixture. The solid was filtered and washed with acetone and dried, to yield 0.85 parts. The structure was confirmed on the basis of ¹H NMR. The reaction proceeded as follows: 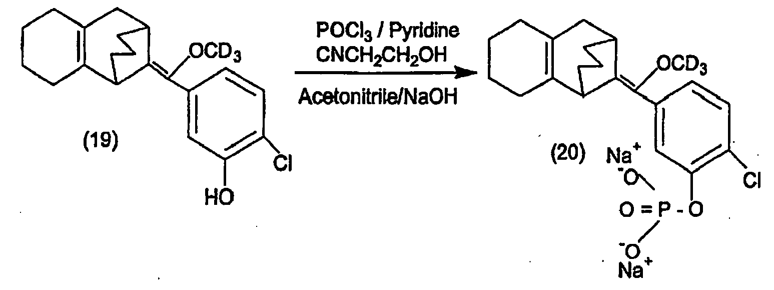
(h). Photooxidation of (3-phosphoryloxyphenyl) methoxy (D₃) methylene tricyclo [7.3.1.0 ^{2,7}] tridec-2,7-ene, disodium salt (21).
   Alkene (20) was photooxidized to give [(4 -Methoxy (D₃)-4-(3-phosphoryloxyphenyl)] Spiro [1,2-dioxetane-3, 13-tricyclo [7.3.1.0^{2,7}] tridec-2,7-ene], disodium salt (21).

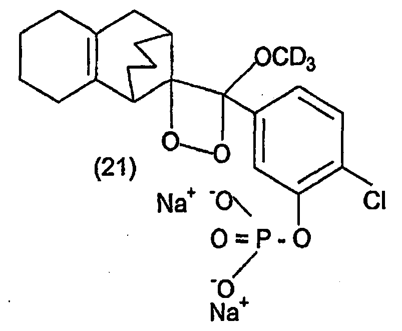

### Example II

This example illustrates the preparation of [4-(2-methoxy(D₃))-4-(3-phosphoryloxy-4-chlorophenyl)] spiro [1, 2-dioxetane-3, 2'-(5-chloroadamantane)], disodium salt (28).
(a). Synthesis of 5-Hydroxyadamantan-2-one (23), (a starting intermediate ketone).
   Into a single neck 500mL round bottom flask equipped with a magnetic stirrer and water bath, 75 parts of acetic acid containing 5 parts acetic anhydride was added. Twenty-five parts of chromium trioxide was added in portions in 40 minutes while the temperature was maintained at 15-20° C with water bath. Five parts of adamantan-2-one (22) was added in portions over a period of 15 minutes. Stirring was continued for one hour. The viscous reaction mixture was poured into cold 250mL of aqueous 20% sodium hydroxide solution. The aqueous layer was extracted with 3x 250mL of ethyl acetate and washed with 2 x 250mL of water and dried over sodium sulfate. Solvent was evaporated under reduced pressure and chromatographed on silica gel column using 75% ethyl acetate / hexane. The desired fractions were collected and solvent was evaporated to give a solid, yield 2.6 parts, single spot on silica gel TLC plate. The structure was confirmed on the basis of ¹H NMR. The reaction proceeded as follows: 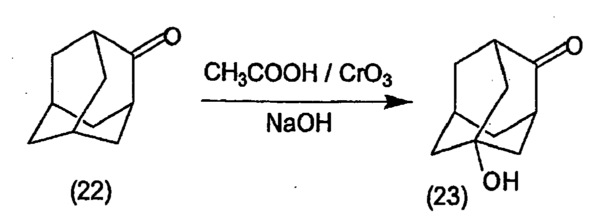
(b). Synthesis of 5-chloroadamantan-2-one (24), (a coupling ketone).
   In a round bottom flask 20 parts of 5-hydroxyadamantan-2-one was dissolved in 120mL of thionylchloride and boiled under refluxed for two hours. The excess thionyl chloride was evaporated. The residue was dissolved in 100mL of methylene chloride and washed with 0.5N sodium hydroxide and then 10% aqueous sodium chloride solution. The organic layer was dried on sodium sulfate and the solvent evaporated under reduced pressure to give a white solid, yield 16.9g. The reaction proceed as follows: 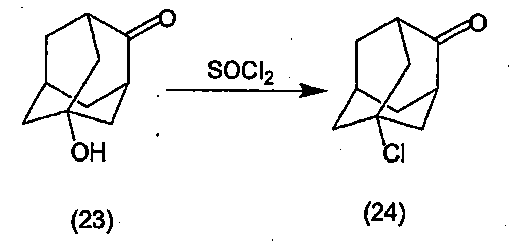
(c). Synthesis of [(3-tert-Butyldimethytsiloxy-4-chtorophenyl) (2-methoxy (D₃) methylene]-5-chloroadamantane (25), (a coupled intermediate alkene).
   Into a 1000mL three-neck flask equipped with magnetic stirrer, pressure-equalizing addition funnel and nitrogen line was charged with 350mL of anhydrous THF. Thirty parts of titanium tetrachloride was added dropwise over a period of 50 minutes. The suspension was stirred for 20 minutes and 43 parts of zinc was added in small portions. The reaction mixture was heated under reflux for 2 hours and 30mL of triethylamine was added dropwise. After refluxing one hour, 7 parts of a solution of methyl (D₃) 3-tert-butyldimethylsiloxy-4-chlorobenzoate and 3 parts of ketone (24) in 60mL of dry THF was added over a period of 75 minutes and the reaction mixture was heated for one hour. The mixture on TLC silia gel plate showed the presence of the starting ester. An additional 2.5 parts of starting ketone (24) in 35mL of dry THF was added dropwise over 90 minutes and refluxed for two hours. The mixture was cooled to room temperature, diluted with 750mL of hexane and decanted. The residue was washed with 3 x 250mL of hexane. The combined hexane layer was filtered and evaporated under reduced pressure to give an oily material which was purified by chromatography on silica gel column using 2.5% ethyl acetate / hexane as an eluant. The fractions were checked by TLC on silica gel plate and the desired fractions were combined. The solvent was evaporated under reduced pressure to give an oil, yield 4.50 parts. The structure was confirmed by ¹H NMR. The reaction proceeded as follows: 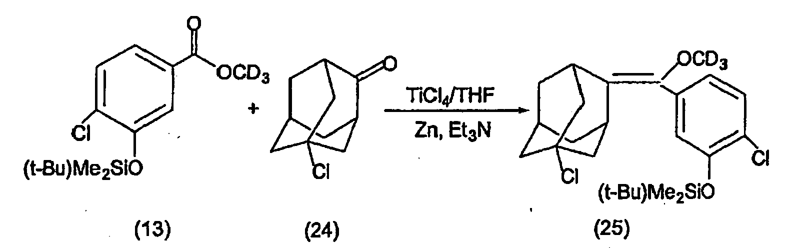
(d). Synthesis of [(3-hydroxy-4-chlorophenyl)(2-methoxy (D₃) methylene]-5-chloroadamantane (26), (alkene).
   To a solution of 4 parts of (3-tert-Butyldimethylsiloxyphenyl) methoxy (D₃) methylene tricyclo[7.3.1.0^{2,7}] tridec-2,7-ene in 100mL of THF was added 5 parts of 70% tetra-n-butylammonium fluoride in 50mL of THF over a period of 10 minutes and stirring was continued for two hours. TLC on silica gel plate showed the formation of new product. Solvent was evaporated dnder reduced pressure and the oily material was dissolved in 250mL of methylene chloride and washed with 2 x 100mL of water. After drying over anhydrous sodium sulfate, the solvent was evaporated and the oily product was purified on silica gel column. TLC on silica gel plate of the fractions were checked and the combined organic solvent was evaporated, yield 2.25 parts. The structure of the product was confirmed on the basis of ¹ H NMR. The reaction proceeded as follows: 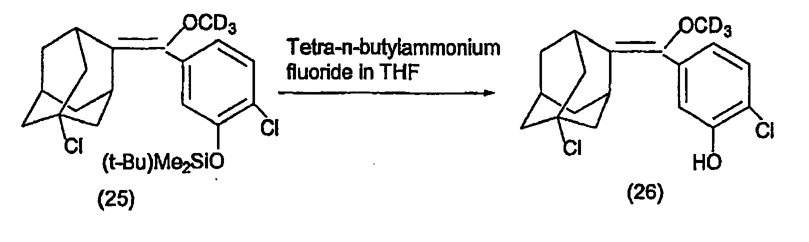
(e). Synthesis of [(3-phosphoryloxy-4-chlorophenyl)(2-methoxy (D₃) methylene]-5-chloroadamantane, disodium salt (27).
   Into a 250mL three-neck round bottom flask equipped with magenetic stirrer was added 20 mL of dry THF under nitrogen and then 1.45mL of phosphorous oxychioride was added drop-wise (reaction flask was cooled in ice-water bath). A solution of 1.45 part of alkene (26) in 15mL of THF containing 0.7 parts of anhydrous pyridine was added to the reaction flask over a period of 30 minutes. The reaction mixture was stirred at room temperature for 3 hours. TLC on silica gel plate showed the formation of new product. The solvent was evaporated to dryness and 10mL of THF was added to the reaction flask. A solution containing 0.95 parts of 3-hydroxypropionitrile and 1.0 parts of anhydrous pyridine in 7.5ml of dry THF was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column using 70% ethyl acetate /hexane containing 0.1 % triethylamine. Fractions were checked by TLC on silica gel plate and the desired fractions were combined and evaporated under reduced pressure to give an oil, yield 1.40 parts. The oily material was dissolved in 15mL of dry THF and a solution of 1.0 parts of sodium hydroxide in 7mL of water was added drowise. Stirring was continued for two hours and the reaction mixture was diluted with 15mL of acetonitrile. The solid was filtered and washed with acetonitrile. The solid material was crystallised with methanol and acetone mixture. The solid was filtered and washed with acetone and dried, to yield 1.2 parts. The structure was confirmed on the basis of ¹ H NMR. The reaction proceeded as follows: 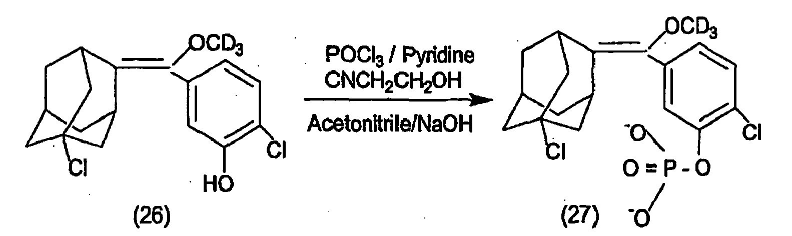
(f). Photo-oxidation of [(3-phosphoryloxy-4-chlorophenyl)(2-methoxy (D₃) methylene]-5-chloroadamantane, disodium salt (27).
   Alkene (27) was photooxldised as reported above, to give [4-(2-methoxy (D₃))-4-(3-phosphoryloxy-4-chlorophenyl)] spiro [1,2-dioxetane-3, 2'-(5-chloroadamantane)], disodium salt (28).

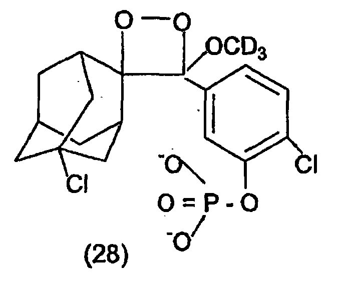

### Example III

This example illustrates the preparation of [4-methoxy (D3)-4-phosphoryloxy-4-chlorophenyl)] spiro [1, 2-dioxetane-3, 2'-adamantan-4, 5-ene], disodium salt (33). The sequence of the reactions in accordance herewith:
(a). Synthesis of adamanten-2-one-4,5-ene (29), (coupling ketone).
   In a 1000mL round bottom flask 50 parts of 5-hydroxy-2-adamantanone was dissolved in warm benzene (400ml). The solution was heated at reflux for 6 hours in the presence of 7mL of concentrated sulfuric acid with Dean-Stark water separator. Additional 5mL concentrated sulfuric acid added and heating continued for additional 18 hours. The reaction mixture was cooled to room temperature and benzene layer was decanted off. The organic layer was washed with deionized water (100ml) and dried over anhydrous sodium sulfate. The solvent was evaporated under reduced pressure and purified on silica gel column eluting with 20% ethyl acetate-hexanes. Fractions were checked by TLC on silica gel plates and product fractions were combined. The solvent was evaporated to give 31.2 parts of title compound as solid. The structure was confirmed by ¹H NMR. The reaction proceeded as follows: 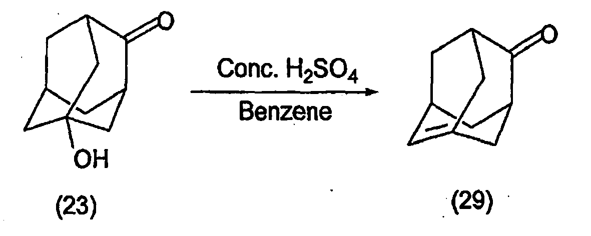
(b). Synthesis of [(3-t-butyldimethylsilyloxy 4-chlorophenyl) (2-methoxy (D₃)) methylene]-4, 5-adamantene (30), (a coupled intermediate alkene).
   Into a 3000mL three-neck flask equipped with mechanical stirrer, pressure-equalizing addition funnel and nitrogen line was charged with 200mL of anhydrous THF. Fourty one parts of titanium tetrachloride was added dropwise over a period of 30 minutes. The suspension was stirred for 20 minutes and 34 parts of zinc was added in small portions over 20 minutes. The reaction mixture was heated under reflux for 4 hours and 85mL of triethylamine was added dropwise over 15 minutes.
   After refluxing one hour, 9.5 parts of a solution of methyl (D₃) 3-tert-butyldimethylsiloxybenzoate (13) and 5.44 parts of adamanten-2-one-4,5-ene (29) in 100mL of dry THF was added over a period of 90 minutes and the reaction mixture was heated for one hour. The mixture on TLC silica gel plate showed the presence of the starting ester. An additional 0.67 parts of adamanten-2-one-4,5-ene (29) in 25mL of dry THF was added dropwise over 30 minutes and refluxed for two hours. The mixture was cooled to room temperature, diluted with 500mL of hexane and decanted. The residue was washed with 3 x 200mL of hexane. The combined hexane layer was filtered and evaporated under reduced pressure to give an oily material which was purified by chromatography on silica gel column eluting with 2.5% ethyl acetate-hexanes with 0.25% triethylamine. Fractions were checked by TLC on silica gel plate and product containing fractions were combined. The solvent was evaporated under reduced pressure gave 8.9 parts of title compound (30) as an oil. The structure was confirmed by ¹H NMR. The reaction proceeded as follows: 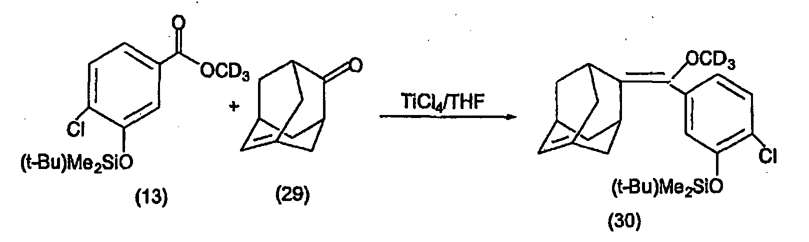
(c). Synthesis of [(3-hydroxy-4-chlorophenyl)-(2-methoxy (D₃)) methylene]-4,5-adamantene (31), (alkene)
   A solution of 8.9 parts of alkene (30) in 100mL of THF was added 7.4 parts of 75 Wt% tetra-n-butylammonium fluoride in 50mL of THF over a period of 10 minutes and stirring was continued for 2 hours. TLC on silica gel plate showed the formation of new product. Solvent was evaporated under reduced pressure and the oily material was dissolved in 150mL of methylene chloride and washed with 2 x 100mL of deionized water. After drying over anhydrous sodium sulfate, the solvent was evaporated and the oily product was purified on silica gel column eluting with 15% ethyl acetate-hexanes with 0.2%triethylamine. Fractions were checked on silica gel TLC plate and product-containing fractions were combined. The solvent was evaporated under reduced pressure, gave 6.35 parts title compound (31) as thick oil. The structure of the product was confirmed on the basis of ¹H NMR. The reaction proceeded as follows: 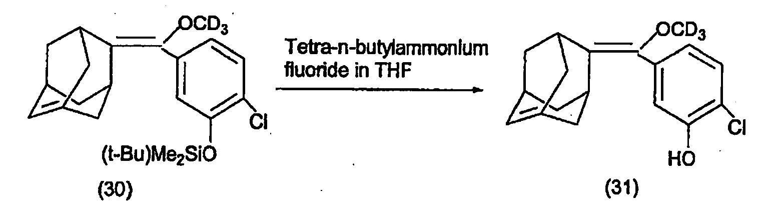
(d). Synthesis of [(3-phosphoryloxy-4-chlorophenyl) methoxy (D₃)) methylene]-4,5-adamantene, disodium salt (32).
   Into a 1000mL three-neck round bottom flask equippd with magenetic stirrer was added 125mL of dry THF under nitrogen and then 5.8mL of phosphorous oxychloride was added dropwise over 25 minutes (reaction flask was cooled in ice-water bath). A solution of 6.3 parts of alkene (31) in 150mL of THF containing 17.4 parts of anhydrous pyridine was added to the reaction flask over a period of 120 minutes. The reaction mixture was stirred at room temperature for 3 hours. TLC on silica gel plate showed the formation of new product. The solvent was evaporated to dryness and 150mL of THF was added to the reaction flask. A solution containing 11.4 parts of 3-hydroxypropionitrile and 12.8 parts of anhydrous pyridine in 100ml of dry THF was added dropwise to the reaction mixture over 40 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1 % triethylamine. Fractions were checked by TLC on silica-gel plate and the desired fractions were combined and evaporated under reduced pressure to give an oil, 5.8 parts. The structure was confirmed on the basis of ¹H NMR. The oily material was dissolved in 75mL of dry THF and a solution of 1.2 parts of sodium hydroxide in 7mL of water was added dropwise. Stirring was continued for two hours and the reaction mixture was diluted with 10mL of acetonitrile. The solid was filtered and washed with acetonitrile. The solid material was crystallized with a methanol and acetone mixture. The solid was filtered and washed with acetone and dried, to yield 4.68 parts. The structure was confirmed on the basis of ¹ H NMR. The reaction proceeded as follows: 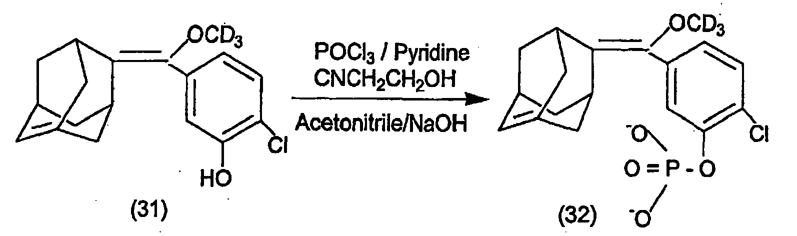
(e). Photooxidation of [(3-phosphoryloxy-4-chlorophenyl) methoxy (D₃)) methylene]-4,5-adamantene, disodium salt (32).
   Alkene (32) was photooxidised as reported above, to give [4-methoxy (D₃)-4-phosphoryloxy-4-chlorophenyl)] spiro [1,2-dioxetane-3, 2'-adamantan-4, 5-ene], disodium salt (33).

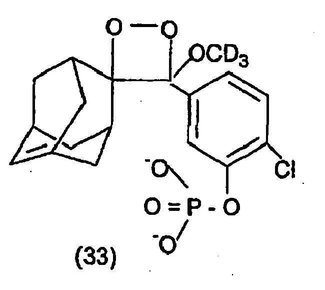

### Example IV

This example illustrates the preparation of [(4-Methoxy (D3))-4-(3-phosphoryloxy-4-chlorophenyl)] spiro [1,2-dioxetane-3, 2'-adamantane], disodium salt (37). The sequence of the reactions in accordance herewith:
(a). Synthesis of [(3-t-butyldimethylsilyloxy-4-chlorophenyl) (2-methoxy (D3) methylene]-adamantane (34), (a couped intermediate alkene).
   Into a 3000mL three-neck flask equipped with mechanical stirrer, pressure-equalizing addition funnel and nitrogen line was charged with 350mL of anhydrous THF 79.5 parts of titanium tetrachloride was added dropwise over a period of 30 minutes. The suspension was stirred for 20 minutes and 63.9 parts of zinc was added in small portions over 20 minutes. The reaction mixture was heated under reflux for 4 hours and 140mL of triethylamine was added dropwise over 15 minutes. After refluxing one hour, 15.6 parts of asolution of methyl (D₃) 3-tert-butyldimethylsiloxybenzoate (13) and 8 parts of 2-adamantanone (22) in 125mL of dry THF was added over a period of 125 minutes and the reaction mixture was heated for one hour. The mixture on TLC silica gel plate showed the presence of the starting ester. An additional 2 parts of 2-adamantanone (22) in 35mL of dry THF was added dropwise over 90 minutes and refluxed for two hours. The mixture was cooled to room temperature, diluted with 500mL of hexane and decanted. The residue was washed with 3 x 200mL of hexane. The combined hexane layer was filtered and evaporated under reduced pressure to give an oily material which was purified by chromatography on silica gel column eluting with 2.5% ethyl acetate-hexanes with 0.25% triethylamine. Fractions were checked by TLC on silica gel plate & product containing fractions were combined. The solvent was evaporated under reduced pressure gave 18.6 parts of title compound (34) as an oil. The structure was confirmed by ¹H NMR. The reaction proceeded as follows: 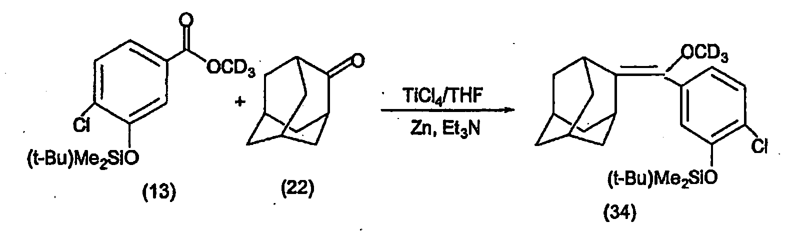
(b). Synthesis of [(3-hydroxy-4-chlorophenyl)-(2-methoxy (D₃)) methylene]-adamantane (35), (alkene).
   To a solution of 18.6 parts of purified silylalkene (34) in 150mL of THF was added 15.4 parts of 75 Wt% tetra-n-butylammonium fluoride in 50mL of THF over a period of 10 minutes and stirring was continued for 2 hours. TLC on silica gel plate showed the formation of new product. Solvent was evaporated under reduced pressure and the oily material was dissolved in dichloromethane (300mL) and washed with deionized water (2x150mL). After drying over anhydrous sodium sulfate, the solvent was evaporated and the oily product was purified on silica gel column eluting with 15% ethyl acetate-hexanes with 0.2%triethylamine. Fractions were checked on silica gel TLC plate and product-containing fractions were combined. The solvent was evaporated under reduced pressure gave 13.26 parts title compound (35). The structure of the product was confirmed on the basis of ¹H NMR. The reaction proceeded as follows: 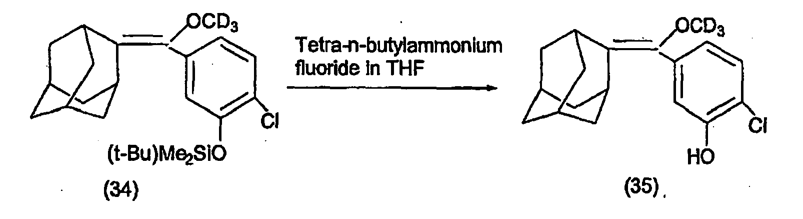
(c). Synthesis of [(3-phosphoryloxy-4-chlorophenyl) methoxy (D₃)) methylene]-adamantane, disodium salt (36).
   Into a 1000mL three-neck round bottom flask equippd with magenetic stirrer was added 150mL of dry THF under nitrogen and then 11.8mL of phosphorous oxychloride was added dropwise over 25 minutes (reaction flask was cooled in Ice-water bath). A solution of 13.1 parts of alkene (35) in 150mL of THF containing 35 parts of anhydrous pyridine was added to the reaction flask over a period of 90 minutes. The reaction mixture was stirred at room temperature for 3 hours. TLC on silica gel plate showed the formation of new product The solvent was evaporated to dryness and 150 mL of THF was added to the reaction flask. A solution containing 22.65 parts of 3-hydroxypropionitrile and 24.9parts of anhydrous pyridine in 100ml of dry THF was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1% triethylamine. Fractions were checked by TLC on silica-gel plate and the desired fractions were combined and evaporated under reduced pressure to give an oil, 12.94 parts. The structure was confirmed on the basis of ¹H NMR. The oily material was dissolved in 125mL of dry THF and a solution of 2.6 parts of sodium hydroxide in 10mL of water was added dropwise. Stirring was continued for two hours and the reaction mixture was diluted with 10mL of acetonitrile. The solid was filtered and washed with acetonitrile. The solid material was crystallized with a methanol and acetone mixture. The solid was filtered and washed with acetone and dried, to yield 10.2 parts. The structure was confirmed on the basis of ¹H NMR. The reaction proceeded as follows: 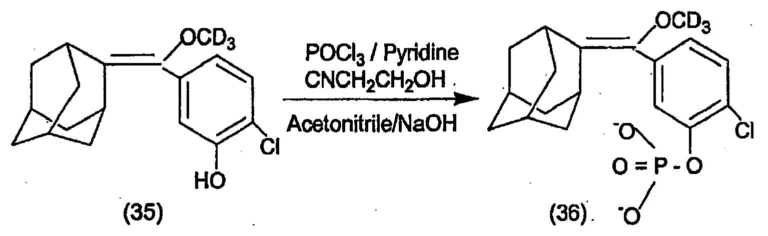
(d). Photooxidation of [(3-phosphoryloxy-4-chlorophenyl) methoxy (D₃)) methylene]-adamantane, disodium salt (36).
   Alkene (36) was photooxidised as reported above, to give [(4-Methoxy (D3)-4-(3-phosphoryloxy-4-chlorophenyl)] spiro [1, 2-dioxetane-3, 2'-adamantane], disodium salt (37).

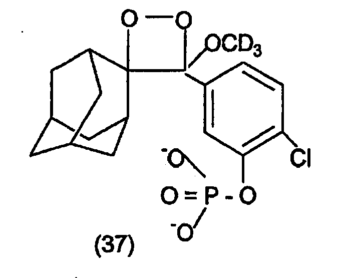

### Example V

This example illustrates the preparation of [4-Methoxy (D₃)-4-(3-phosphoryloxyphenyl)] spiro [1, 2-dioxetane-3, 2'-admantane], disodium salt (44). The sequence of the reactions in accordance herewith:
(a). Synthesis of Methyl (D₃) 3-hydroxybenzoate (39), (a starting intermediate ester).
   Into a 250mL round bottom flask equipped with magnetic stirrer and heating mantle was added 15 parts of 3-hydroxybenzoic acid (38), deuterated methanol (65mL) and concentrated sulfuric acid (1 mL). The reaction mixture was heated under gentle reflux for 22 hours. The solvent was evaporated under reduced pressure and the residue was dissolved in ethyl acetate (150 ml). The organic layer was washed with deionized water (250mL), 5% aqueous sodium bicarbonate solution (250mL) and deionized water (250mL). The ethyl acetate layer was dried over anhydrous sodium sulfate and the solvent was evaporated under reduced pressureto give oily residue, which was purified by chromatography on silica gel column eluting with 20% ethyl acetate-hexanes. Product fractions were checked by TLC on silica gel plates and combined. The solvent was evaporated under reduced pressure and residue stirred with hexanes (50mL) for 30 minutes. The solid was collected by filtration and dreid to give 13.5 parts of the title comound (39). TLC on silica gel plate showed a single spot. The structure of the compound was confirmed on the basis of ¹H NMR. The material was prepared according to the following equation: 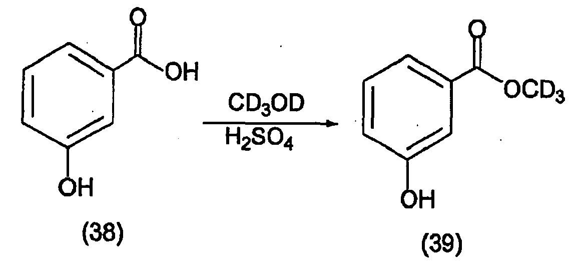
(b). Synthesis of methyl 3-tert-butyldimethylsiloxybenzoate (40), (a coupling ester).
   In to a 250mL round bottom flask, dry dimethylformamide (50mL), 13 parts of the deuteromethyl ester (39) and 15.9 parts of tert-butyldimethylsilyi chloride were added with stirring under nitrogen atmosphere. 15.1 parts of imidazole was added in portions and stirring was continued for 5 hours. The reaction mixture was extracted with hexanes (3 x 300mL) and the hexane layer was washed with deionized water (2x250mL). The organic layer was dried over anhydrous sodium sulfate and the solvent was evaporated under reduced pressure to give 22 parts of the title compound (40) as oil. This material was pure by TLC on silica gel plates and the structure was confirmed by ¹H NMR. The material was prepared according to the following equation. 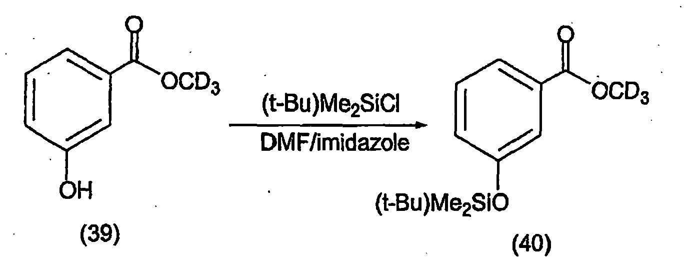
(c). Synthesis of [(3-t-butyldimethylsilyloxyphenyl) (2-methoxy (D3) methylene]-adamantane (41), (a coupled intermediate alkene).
   Into a 3000mL three-neck flask equipped with mechanical stirrer, pressure-equalizing addition funnel and nitrogen line was charged with anhydrous THF (475mL). One-hundered six parts of titanium tetrachloride was added dropwise over a period of 25 minutes. The suspension was stirred for 20 minutes and 88.2 parts of zinc was added in small portions over 20 minutes. The reaction mixture was heated under reflux for 4 hours and 150mL of triethylamine was added dropwise over 40 minutes. After refluxing one hour, a solution of 21.8 parts methyl (D₃) 3-tert-butyldimethyl siloxybenzoate (40) and 13.9 parts of 2-adamantanone (22) in anhydrous THF (125mL) was added over a period of 105 minutes and the reaction mixture was heated for one hour. The mixture on TLC silica gel plate showed the presence of the starting ester. An additional 1.39 parts of 2-adamantanone (22) in 25mL of dry THF was added dropwise over 30 minutes and refluxed for 4 hours. The mixture was cooled to room temperature, diluted with 500mL of hexane and decanted. The residue was washed with hexanes (3x200mL). The combined hexanes layer was filtered and evaporated under reduced pressure to give an oily material, which was purified by chromatography on silica gel column eluting with 2.5% ethyl acetate-hexanes with 0.25% triethylamine. Fractions were checked by TLC on silica gel plate & product containing fractions were combined. The solvent was evaporated under reduced pressure gave 17.8 parts of title compound (41) as an oil. The structure was confirmed by ¹H NMR. The reaction proceeded as follows: 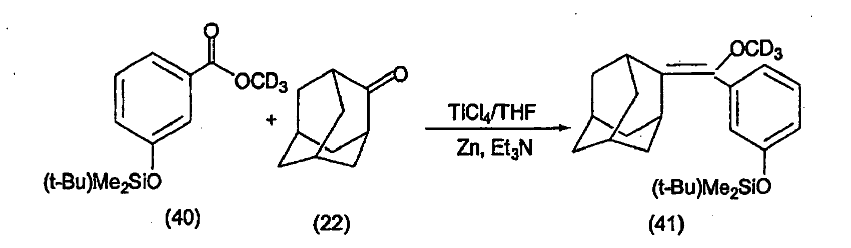
(d). Synthesis of [(3-hydroxyphehyl)-(2-methoxy (D₃)) methylene]-adamantane (42), (alkene).
   To a solution 17.65 parts of purified silylalkene (41) in THF (250mL) was added 16.1 parts of 75 wt% tetra-n-butylammonium fluoride in THF (50mL) over a period of 10 minutes and stirring was continued for 2 hours. TLC on silica gel plate showed the formation of new product. Solvent was evaporated under reduced pressure and the oily material was dissolved in dichloromethane (300mL) and washed with deionized water (2x150mL). After drying over anhydrous sodium sulfate, the solvent was evaporated and the oily product was purified on silica gel column eluting with 15% ethyl acetate-hexanes containing 0.25% triethylamine. Fractions were checked on silica gel TLC plate and product-containing fractions were combined. The solvent was evaporated under reduced pressure gave 11.96 parts title compound (42) The structure of the product was confirmed on the basis of ¹H NMR. The reaction proceeded as follows: 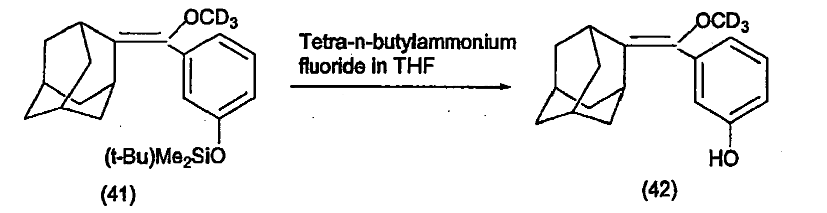
(e). Synthesis of [(3-phosphoryloxyphenyl) methoxy (D₃) methylene]-adamantane, disodium salt (43).
   Into a 1000mL three-neck round bottom flask equippd with magenetic stirrer was added dry THF (75mL) under nitrogen and then 6.35 ml of phosphorous oxychloride was added dropwise over 25 minutes (reaction flask was cooled in ice-water bath). A solution of 6.1 parts of alkene (41) in THF (100mL) containing 18.9 parts of anhydrous pyridine was added to the reaction flask over a period of 90 minutes. The reaction mixture was stirred at room temperature for 3 hours. TLC on silica gel plate showed the formation of new product. The solvent was evaporated to dryness and fresh THF (150mL) was added to the reaction flask containing 12.9 parts of 3-hydroxypropionitrile and 14.4 parts of anhydrous pyridine in dry THF (75mL) was added dropwise to the reaction mixture over 25 minutes and was stirred for 15 hours at room temperature. The reaction was cooled to ice-water temperature and the solid was filtered arid washed with cold THF. The solvent was evaporated and oily material was chromatographed on silica gel column eluting with 75% ethyl acetate-hexane containing 0.1 % triethylamine, Fractions were checked by TLC on silica gel plates and the product containing fractions were combined. The solvent was evaporated under reduced pressure gave 7.75 parts phosphate ester. The structure was confirmed on the basis of ¹H NMR. A solution of 1.75 parts of sodium hydroxide in water (8mL) was added dropwise to a solution of phosphate ester in THF (75 ml) over 20 minutes and stirred for 2 hours. The reaction mixture was diluted with acetonitrile (15mL). The solid was filtered and washed with acetonitrile. The solid material was crystallized with a methanol and acetone mixture. The solid was filtered, washed with acetone and dried gave 6.2 parts of the title compound (43). The structure was confirmed on the basis of ¹H NMR. The reaction proceeded as follows: 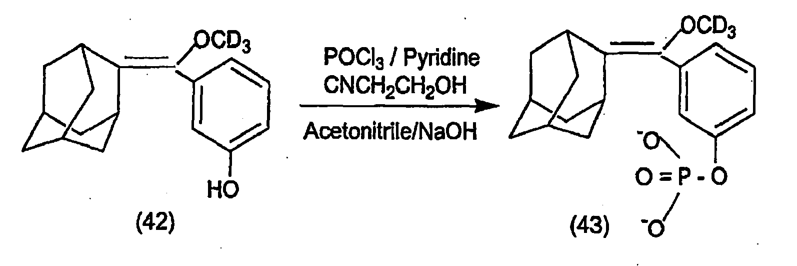
(f). Photooxidation of [(3-phosphoryloxyphenyl) methoxy (D₃)) methylene]-adamantane, disodium salt (43).
   Alkene (43) was photooxidised as reported above, to give [(4-Methoxy (D3)-4-(3-phosphoryloxy-4-chlorophenyl)] spiro [1, 2-dioxetane-3, 2'-adamantane], disodium salt (44).

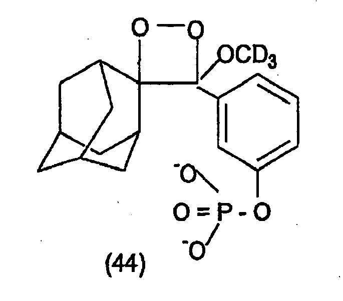

### Example VI

This example illustrates the preparation of [(4-Methoxy (D₃)-4-(3-phosphoryloxy-4-chlorophenyl)] spiro [1, 2-dioxetane-3, 2'-(5-methoxyadamantane)], disodium salt.
(a). Synthesis of 5-Methoxy-2-adamantanone (45), (a coupling ketone).
   In 1000mL three-neck flask with magnetic srirrer, 9.9 parts of sodium hydride, 60% dispersion in mineral oil was mixed with hexanes (150mL) under nitrogen atmosphere and stirred for 5 minutes. Stirring stopped for 10 minutes and hexanes decanted off. This process was repeated with fresh hexanes (2x150mL) and suspended in anhydrous THF (400mL). Thirty-three parts of 5-hydroxy-2-adamantanone was added in portions over 30 minutes and stirred for 1 hour. The mixture was cooled to ∼8-10 °C, 85 parts iodomethane added at once and stirred for 4 hours. Additional 41 parts of iodomethane was added and mixture was allowed to warm to room temperature over 18 hours. The solution was concentrated to dryness under reduced pressure and dissolved in ethyl acetate (500mL). The organic layer was washed with deionized water (2x250mL) and dried over anhydrous sodium sulfate. The organic solution was evaporated under resuced pressure and crude product was purified by chromatography on silica gel column using 20% ethyl acetate-hexanes. The fractions were checked by TLC on silica gel plates and product containing fractions were combined. The solvent was evaporated under reduced pressure gave 27.6 parts of title compound (45) as light yellow oil. The structure was confirmed on basis of ¹H NMR. The reaction proceeded as follows: 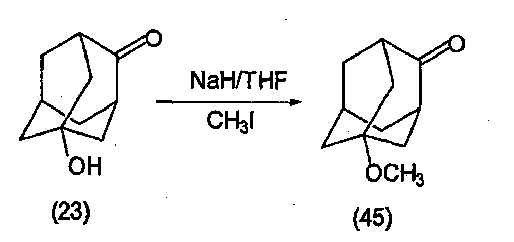
(b). Synthesis of [(3-t-butyldimethylsilyloxy-4-chlorophenyl) (2-methoxy (D3) methylene]-5-methoxyadamantane (46), (a coupled intermediate alkene).
   Into a 2000mL three-neck flask equipped with mechanical stirrer, pressure-equalizing addition funnel and nitrogen line was charged with anhydrous THF (200ml). Fourty two parts of titanium tetrachloride was added dropwise over a period of 15 minutes. The suspension was stirred for 15 minutes and 34.8 parts of zinc was added in small portions over 20 minutes. The reaction mixture was heated under reflux for 4 hours and 85mL of triethylamine was added dropwise over 15 minutes. After refluxing one hour, a solution of 9.25 parts methyl (D₃) 3-tert-butyldimethylsiloxy-4-chlorobenzoate (13) and 5.9 parts of 5-methoxy-2-adamantanone (45) in anhydrous THF (125mL) was added over a period of 85 minutes and the reaction mixture was heated for one hour. The mixture on TLC silica gel plate showed the presence of the starting ester. An additional 1.15 parts of 5-methoxy-2-adamantanone (45) in 25mL of dry THF was added dropwise over 30 minutes and refluxed for 4 hours. The mixture was cooled to room temperature, diluted with 500mL of hexane and decanted. The residue was washed with hexanes (3x200ml). The combined hexanes layer was filtered and evaporated under reduced pressure to give an oily material which was purified by chromatography on silica gel column eluting with 2.5% ethyl acetate-hexanes with 0.25% triethylamine. Fractions were checked by TLC on silica gel plate and product containing fractions were combined. The solvent was evaporated under reduced pressure gave 7.9 parts of title compound (46) as an oil. The structure was confirmed by ¹H NMR. The reaction proceeded as follows: 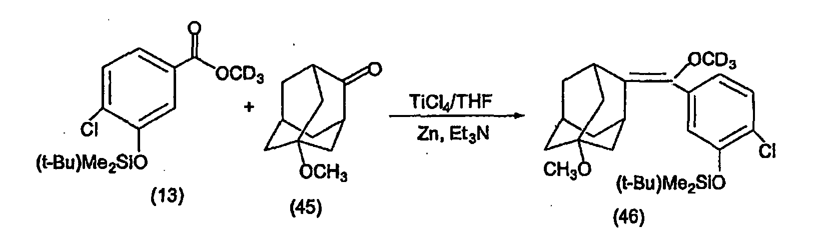
(c). Synthesis of [(3-hydroxy-4-chlorophenyl)-(2-methoxy (D₃)) methylene]-5-methoxyadamantane (47), (alkene).
   To a solution 7.8 parts of purified silylalkene (46) in THF (100mL) was added 6.3 parts of 75 Wt% tetra-n-butylammonium fluoride in THF (30mL) over a period of 10 minutes and stirring was continued for 2 hours. TLC on silica gel plate showed the formation of new product. Solvent was evaporated under reduced pressure and the oily material was dissolved in dichloromethane (200mL) and washed with deionized water (2x100mL). After drying over anhydrous sodium sulfate, the solvent was evaporated and the oily product was purified on silica gel column eluting with 15% ethyl acetate-hexanes containing 0.25% triethylamine. Fractions were checked on silica gel TLC plate and product-containing fractions were combined. The solvent was evaporated under reduced pressure gave 5.55 parts of the title compound (47). The structure of the product was confirmed on the basis of ¹H NMR. The reaction proceeded as follows: 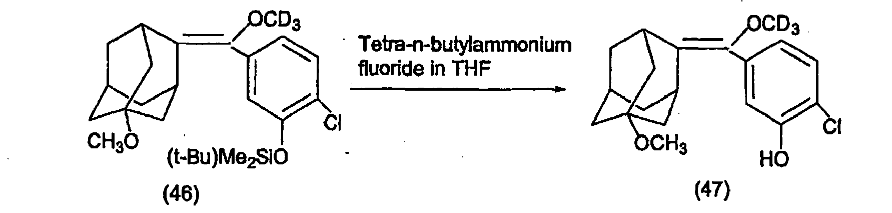
(d). Synthesis of [(3-phosphoryloxy-4-chlorophenyl) methoxy (D₃) methylene]-5-methoxyadamantane, disodium salt (48).
   Into a 1000mL three-neck round bottom flask equippd with magenetic stirrer was added dry THF (100mL) under nitrogen and then 4.65mL of phosphorous oxychloride was added dropwise over 15 minutes (reaction flask was cooled in ice-water bath). A solution of 5.5 parts of alkene (47) in THF (100mL) containing 13.9 parts of anhydrous pyridine was added to the reaction flask over a period of 135 minutes. The reaction mixture was stirred at room temperature for 3 hours. TLC on silica gel plate showed the formation of new product. The solvent was evaporated to dryness and fresh THF (150mL) was added to the reaction flask. A solution containing 9.1 parts of 3-hydroxypropionitrile and 10.4 parts of anhydrous pyridine in dry THF (75mL) was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1% triethylamine. Fractions were checked by TLC on silica gel plates and the product containing fractions were combined. The solvent was evaporated under reduced pressure gave 4.35 parts phosphate ester. The structure was confirmed on the basis of ¹H NMR. A solution of 0.85 parts of sodium hydroxide in water (4 mL) was added dropwise to a solution of phosphate ester in THF (35mL) over 20 minutes and stirred for 2 hours. The reaction mixture was diluted with acetonitrile (10mL). The solid was filtered and washed with acetonitrile. The solid material was crystallized with a methanol and acetone mixture. The solid was filtered, washed with acetone and dried gave 3.6 parts of the title compound (48). The structure was confirmed on the basis of ¹H NMR. The reaction proceeded as follows: 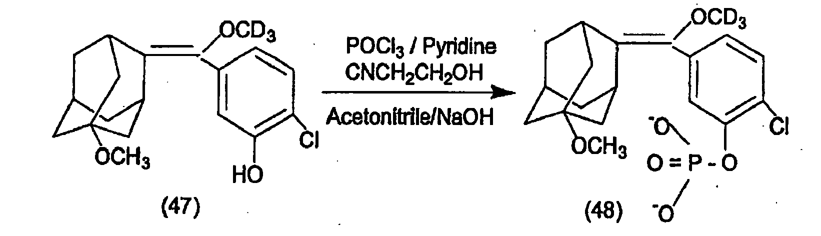
(e). Photooxidation of [(3-phosphoryloxy-4-chlorophenyl) methoxy (D₃) methylene]-5-methoxyadamantane, disodium salt (48).
   Alkene (48) was photooxldised as reported above, to give [(4-Methoxy (D3)-4-(3-phosphoryloxy-4-chlorophenyl)]spiro[1,2-dioxetane-3,2'-adamantane], disodium salt (49).

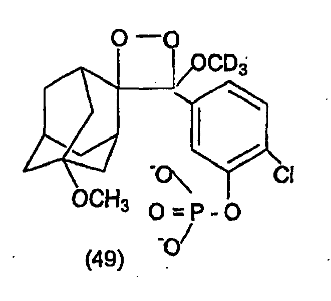

### Example VII

This example illustrate the preparation of [4-Methoxy-4-(3-phosphoryloxy-4-chlorophenyl)] spiro [1, 2-dioxetane-3,2'-(5-methoxy(D₃)admantane)], disodium salt (56). The sequence of the reactions in accordance herewith:
(a). Synthesis of Methyl 3-hydroxy-4-chlorobenzoate (50), (a starting ester).
   In a 250mL round bottom flask, 15 parts of 3-hydroxy-4-chlorobenzoicacid (11, commercially available) was dissolved in methanol (150mL) and concentrated sulfuric acid (2mL). The reaction mixture was heated at reflux for 30 hours. TLC on silica gel plates showed formation of the product The solvent was evaporated under reduced pressure and the residue was dissoved in ethyl acetate (150 ml). The organic layer was washed with 5% aqueous sodium bicarbonate solution (100mL) and deionized water (150mL). The organic sovent was dried over anhydrous sodium sulfate and filtered. The solvent was evaporated under reduced pressure and residue purified by chromatography on silica gel column eluting with 25% ethyl acetate-hexanes. Product containing fractions were checked by TLC on silica gel plates & combined. The solvent was evaporated under reduced pressure gave 14.5 parts of the titile compound (50) as off-white solid. The structure was confirmed by ¹H NMR. The reaction proceeded as follows : 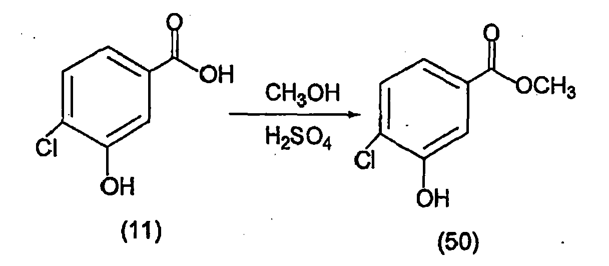
(b). Synthesis of Methyl 3-tert-butyldimethylsilyloxy4-chlorobenzoate (51), (a coupling ester).
   Into a 250mL round bottom flask, dry dimethylformamide (50mL), 14.4 parts of the methylbenzoate (50) and 15 parts of tert-butytdimethylsilyl chloride were added with stirring under nitrogen atmosphere. 13.5 parts of imidazole was added in portions and stirring was continued for 3 hours. The reaction mixture was extracted with hexanes (3 x 300mL) and the hexane layer was washed with deionized water (3x250mL). The organic layer was dried over anhydrous sodium sulfate and the solvent was evaporated under reduced pressure to give 22.4 parts of the title compound (51) as oil. This material was pure by TLC on silica gel plates and the structure was confirmed by ¹H NMR. The material was prepared according to the following equation: 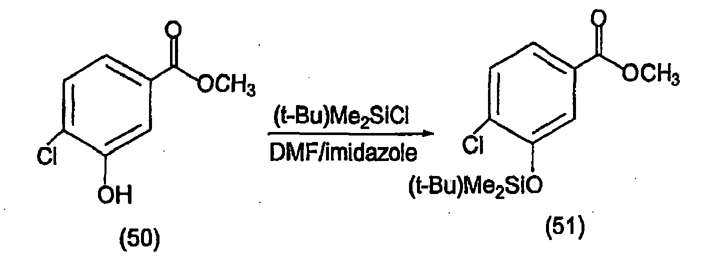
(c). Synthesis of 5-Methoxy (D₃)-2-adamantanone (52), (a coupling ketone).
   In 1000ml three-neck flask with magnetic srirrer, 5.8 parts of sodium hydride (60% dispersion in mineral oil) was mixed with hexanes (125ml) under nitrogen atmosphere and stirred for 3 minutes.
   Stirring stopped for 5 minutes and hexanes decanted off. This process was repeated with fresh hexanes (2x100mL) and suspended in anhydrous THF (250mL). Ninteen parts of 5-hydroxy-2-adamantanone (23) was added in portions over 15 minutes and stirred for 1.25 hours at ∼35^{°} C. Then the mixture was cooled to ∼8-10^{°}C, 25 parts iodomethane-D₃ added at once and allowed to warm to room temperature slowly over 18 hours with stirring. The solution was concentrated to dryness under reduced pressure and dissolved in ethyl acetate (250mL). The organic layer was washed with deionized water (2x200mL) and dried over anhydrous sodium sulfate. The organic solution was evaporated under resuced pressure and crude product was purified by chromatography on silica gel column using 20% ethyl acetate-hexanes. The fractions were checked by TLC on silica gel plates and product containing fractions were combined. The solvent was evaporated under reduced pressure gave 13.2 parts of title compound (52) as light yellow oil. The structure was confirmed on basis of ¹H NMR. The reaction proceeded as follows: 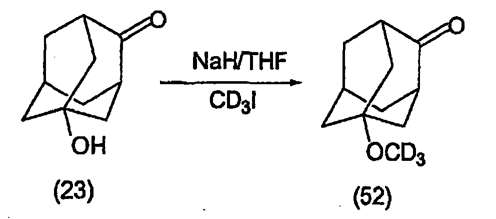
(d). Synthesis of [(3-t-butyldimethylsilyloxy-4-chlorophenyl) (2-methoxy) methylene]-5-methoxy (D₃) adamantane (53), (a coupled intermediate alkene).
   Into a 2000mL three-neck flask equipped with mechanical stirrer, pressure-equalizing addition funnel and nitrogen line was charged with anhydrous THF (250mL). Fourty two parts of titanium tetrachloride was added dropwise over a period of 15 minutes. The suspension was stirred for 15 minutes and 37.8 parts of zinc was added in small portions over 15 minutes. The reaction mixture was heated under reflux for 4 hours and 80mL of triethylamine was added dropwise over 15 minutes. After refluxing one hour, a solution of 7.85 parts methyl 3-tert-butyldimethylsiloxy-4-chlorobenzoate (51) and 5.1 parts of 5-methoxy (D₃)-2-adamantanone (52) in anhydrous THF (125ml) was added over a period of 150 minutes and the reaction mixture was heated for one hour. The mixture on TLC silica gel plate showed the presence of the starting ester. An additional 1.6 parts of 5-methoxy (D₃)-2-adamantanone (52) in anhydrous THF (40mL) was added dropwise over 30 minutes and refluxed for 4 hours. The mixture was cooled to room temperature, diluted with 500mL of hexane and decanted. The residue was washed with hexanes (3x200mL). The combined hexanes layer was filtered and evaporated under reduced pressure to give an oily material, which was purified by chromatography on silica gel column eluting with 2.5% ethyl acetate-hexanes with 0.25% triethylamine. Fractions were checked by TLC on silica gel plate and product containing fractions were combined. The solvent was evaporated under reduced pressure gave 8.3 parts of title compound (53) as an oil. The structure was confirmed by ¹H NMR. The reaction proceeded as follows: 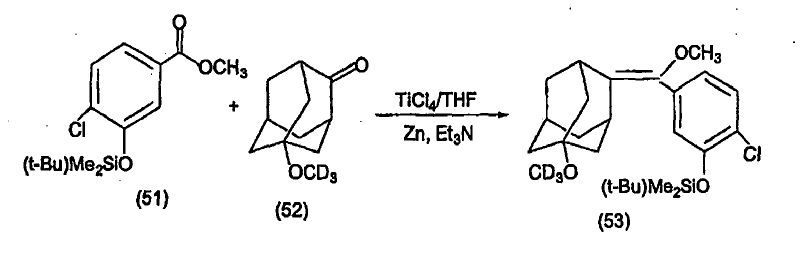
(e). Synthesis of [(3-hydroxy-4-chlorophenyl)-(2-methoxy) methylene]-5-methoxy (D₃) adamantane (54), (alkene).
   To a solution 8.25 parts of purified silylalkene (53) in THF (100ml) was added 6.5 parts of 75 Wt% tetra-n-butylammonium fluoride in THF (30mL) over a period of 10 minutes and stirring was continued for 2 hours. TLC on silica gel plate showed the formation of new product. Solvent was evaporated under reduced pressure and the oily material was dissolved in dichloromethane (200mL) and washed with deionized water (2x100mL). After drying over anhydrous sodium sulfate, the solvent was evaporated and the oily product was purified on silica gel column eluting with 15% ethyl acetate-hexanes containing 0.25% triethylamine. Fractions were checked on silica gel TLC plate and product-containing fractions were combined. The solvent was evaporated under reduced pressure gave 6 parts of the title compound (54). The structure of the product was confirmed on the basis of ¹H NMR. The reaction proceeded as follows: 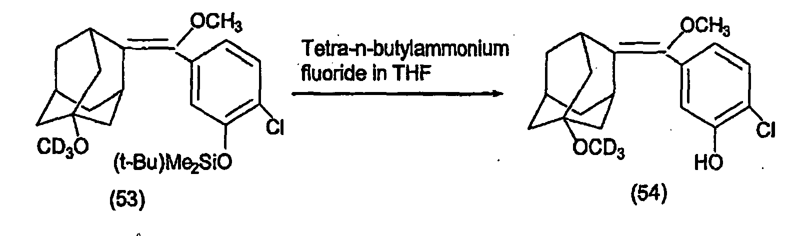
(f). Synthesis of [(3-phosphoryloxy-4-chlorophenyl) (methoxy) methylene]-5-methoxy (D₃) adamantane, disodium salt (55):
   Into a 1 000mL three-neck round bottom flask equippd with magenetic stirrer was added dry THF (100mL) under nitrogen and then 4.9mL of phosphorous oxychloride was added dropwise over 15 minutes (reaction flask was cooled in ice-water bath). A solution of 6 parts of alkene (54) in THF (100mL) containing 14.9 parts of anhydrous pyridine was added to the reaction flask over a period of 130 minutes. The reaction mixture was stirred at room temperature for 3 hours. TLC on silica gel plate showed the formation of new product. The solvent was evaporated to dryness and fresh THF (150mL) was added to the reaction flask. A solution containing 9.5 parts of 3-hydroxypropionitrile and 10.6 parts of anhydrous pyridine in dry THF (75mL) was added dropwise to the reaction mixture over 60 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1 % triethylamine. Fractions were checked by TLC on silica gel plates and the product containing fractions were combined. The solvent was evaporated under reduced pressure gave 5.5 parts of phosphate ester. The structure was confirmed on the basis of ¹H NMR. A solution of 1.1 parts of sodium hydroxide in water (4mL) was added dropwise to a solution of phosphate ester in THF (35mL) over 20 minutes & stirred for 2 hours. The reaction mixture was diluted with acetonitrile (10 ml). The solid was filtered and washed with acetonitrile. The solid material was crystallized with a methanol and acetone mixture. The solid was filtered, washed with acetone and dried gave 4.8 parts of the title compound (55). The structure was confirmed on the basis of ¹H NMR. The reaction proceeded as follows: 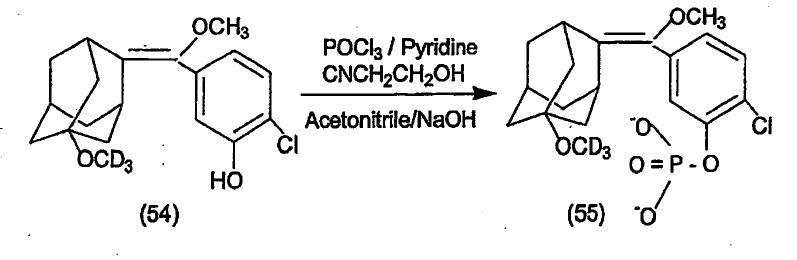
(g). Photooxidation of [(3-phosphoryloxy-4-chlorophenyl) (methoxy) methylene]-5-methoxy (D₃) adamantane, disodium salt (55).
   Alkene (55) was photooxidised as reported above, to give [4-Methoxy-4-(3-phosphoryloxy-4-ch;lorophenyl)] spiro [1,2-dioxetane-3,2'-(5-methoxy (D₃) admantane)], disodium salt (56).

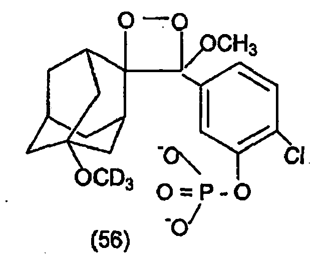

### Example VIII

This example illustrates the preparation of [(4-Methoxy (D₃)-4-(3-phosphoryloxy-4-chlorophenyl)] spiro [1,2-dioxetane-3, 2'-(5-methoxy D₃) adamantane)], disodium salt (60). The sequence of the reaction in accordance herewith:
(a). Synthesis of [(3-t-butyldimethylsilyloxy-4-chlorophenyl) (2-methoxy (D3) methylene]-5-methoxy (D₃) adamantane (57), (a coupled intermediate alkene).
   Into a 2000mL three-neck flask equipped with mechanical stirrer, pressure-equalizing addition funnel and nitrogen line was charged with anhydrous THF (225ml). A fourty one part of titanium tetrachloride was added dropwise over a period of 15 minutes. The suspension was stirred for 15 minutes and 36.7 parts of zinc was added in small portions over 20 minutes. The reaction mixture was heated under reflux for 4 hours and 80mL of triethylamine was added dropwise over 15 minutes. After refluxing one hour, a solution of 8.3 parts methyl (D₃) 3-tert-butyldimethylsiloxy-4-chlorobenzoate (13) and 5.1 parts of 5-methoxy (D₃)-2-adamantanone (52) in anhydrous THF (125mL) was added over a period of 90 minutes and the reaction mixture was heated for one hour. The mixture on TLC silica gel plate showed the presence of the starting ester. An additional 1.34 parts of 5-methoxy (D₃)-2-adamantanone (52) in anhydrous THF (25 ml) was added dropwise over 45 minutes and refluxed for 4 hours. The mixture was cooled to room temperature, diluted with 500 mL of hexane and decanted. The residue was washed with hexanes (3x200mL). The combined hexanes layer was filtered and evaporated under reduced pressure to give an oily material, which was purified by chromatography on silica gel column eluting with 2.5% ethyl acetate-hexanes with 0.25% triethylamine. Fractions were checked by TLC on silica gel plate and product containing fractions were combined. The solvent was evaporated under reduced pressure gave 5.5 parts of title compound (57) as oil. ¹H NMR confirmed the structure. The reaction proceeded as follows: 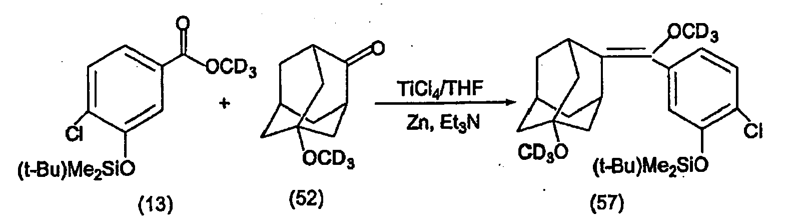
(b). Synthesis of [(3-hydroxy-4-chlorophenyly(2-methoxy (D₃)) methylene]-5-methoxy (D₃) adamantane (58), (alkene).
   To a solution 5.5 parts of purified silylalkene (57) in THF (100mL) was added 4.3 parts of 75 Wt% tetra-n-butylammonium fluoride in THF (30mL) over a period of 10 minutes and stirring was continued for 1.5 hours. TLC on silica gel plate showed the formation of new product. Solvent was evaporated under reduced pressure and the oily material was dissolved in dichloromethane (150 ml) and washed with deionized water (2x100mL). After drying over anhydrous sodium sulfate, the solvent was evaporated and the oily product was purified on silica gel column eluting with 15% ethyl acetate-hexanes containing 0.25% triethylamine. Fractions were checked on silica gel TLC plate and product-containing fractions were combined. The solvent was evaporated under reduced pressure gave 3.9 parts of the title compound (58). The structure of the product was confirmed on the basis of ¹H NMR. The reaction proceeded as follows: 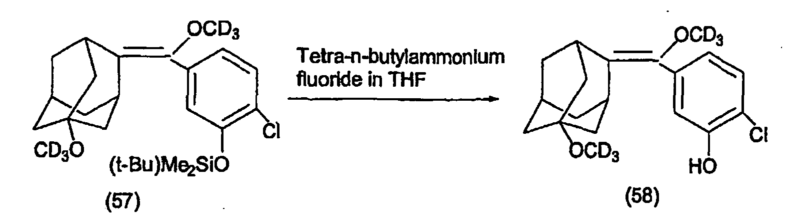
(c). Synthesis of [(3-phosphoryloxy-4-chlorophenyl) (methoxy (D₃) methylene]-5-methoxy (D₃) adamantane, disodium salt (59):
   Into a 1000mL three-neck round bottom flask equippd with magenetic stirrer was added dry THF (100mL) under nitrogen and then 3.3mL of phosphorous oxychloride was added dropwise over 15 minutes (reaction flask was cooled in ice-water bath). A solution of 3.8 parts of alkene (58) in THF (100mL) containing 9.7 parts of anhydrous pyridine was added to the reaction flask over a period of 150 minutes. The reaction mixture was stirred at room temperature for 3 hours. TLC on silica gel plate showed the formation of new product. The solvent was evaporated to dryness and fresh THF (150mL) was added to the reaction flask. A solution containing 6.1 parts of 3-hydroxypropionitrile and 6.9 parts of anhydrous pyridine in dry THF (50mL) was added dropwise to the reaction mixture over 35 minutes and was stirred for 20 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1% triethylamine. Fractions were checked by TLC on silica gel plates and the product containing fractions were combined. The solvent was evaporated under reduced pressure gave 4.7 parts of phosphate ester. The structure was confirmed on the basis of ¹H NMR. A solution of 0.95 parts of sodium hydroxide in water (4mL) was added dropwise to a solution of phosphate ester in THF (35mL) over 20 minutes and stirred for 2 hours. The reaction mixture was diluted with acetonitrile (10mL). The solid was filtered and washed with acetonitrile. The solid material was crystallized with a methanol and acetone mixture. The solid was filtered, washed with acetone and dried gave 3.9 parts of the title compound (59). The structure was confirmed on the basis of ¹H NMR. The reaction proceeded as follows: 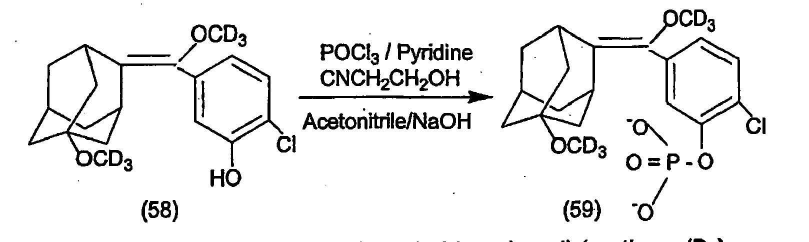
(d). Photooxidation of [(3-phosphoryloxy-4-chlorophenyl) (methoxy (D₃) methylene]-5-methoxy (D₃) adamantane, disodium salt (59).
   Alkene (60) was photooxidised as reported above, to give [(4-Methoxy (D₃)-4-(3-phosphoryloxyphenyl)] spiro [1,2-dioxetane-3, 2'-5-methoxy (D₃) adamantane], disodium salt (61).

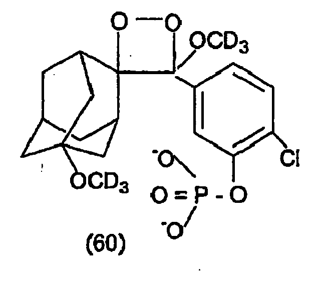

### Example IX

This example illustrates the preparation of [(4-Methoxy (D₃)-4-(3-phosphoryloxyphenyl)] spiro [1,2-dioxetane-3, 2'-(5-chloroadmantane)], disodium salt (64). The sequence of the reaction in accordance herewith:
(a). Synthesis of [(3-tert-butyldimethylsilyloxyphenyl) (2-methoxy (D3)) methylene]-5-chloroadamantane (61), (a coupled intermediate alkene).
   Into a 2000mL three-neck flask equipped with mechanical stirrer, pressure-equalizing addition funnel and nitrogen line was charged with anhydrous THF (250mL). Fourty two parts of titanium tetrachloride was added dropwise over a period of 20 minutes. The suspension was stirred for 20 minutes and 36.4 parts of zinc was added in small portions over 20 minutes. The reaction mixture was heated under reflux for 4.5 hours and 80 mL of triethylamine was added dropwise over 10 minutes. After refluxing one hour, a solution of 8.9 parts methyl (D₃) 3-tert-butyldimethylsiloxybenzoate (40) and 5.6 parts of 5-chloro-2-adamantanone (24) in anhydrous THF (125mL) was added over a period of 95 minutes and the reaction mixture was heated for one hour. The mixture on TLC silica gel plate showed the presence of the starting ester. An additional 1.7 parts of 5-chloro-2-adamantanone (24) in anhydrous THF (25mL) was added dropwise over 30 minutes and refluxed for 4 hours. The mixture was cooled to room temperature, diluted with 500mL of hexane and decanted. The residue was washed with hexanes (3x200mL). The combined hexanes layer was filtered and evaporated under reduced pressure to give an oily material, which was purified by chromatography on silica gel column eluting with 2.5% ethyl acetate-hexanes with 0.25% triethylamine. Fractions were checked by TLC on silica gel plate and product- containing fractions were combined. The solvent was evaporated under reduced pressure gave 5 parts of title compound (61) as an oil. The structure was confirmed, by ¹H NMR. The reaction proceeded as follows: 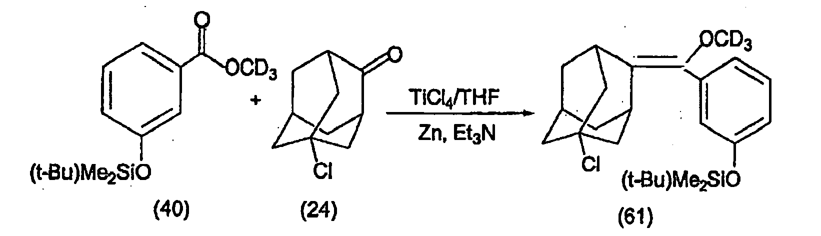
(b). Synthesis of [(3-hydroxyphenyl)-2-methoxy (D₃)) methylene]-5-chloroadamantane (62), (alkene).
   To a solution of 4.95 parts of purified silylalkene (61) in THF (100mL) was added 4.1 parts of 75 Wt% tetra-n-butylammonium fluoride in THF (40 ml) over a period of 10 minutes and stirring was continued for 1.5 hours. TLC on silica gel plate showed the formation of new product. Solvent was evaporated under reduced pressure and the oily material was dissolved in dichloromethane (100 ml) and washed with deionized water (2x75mL). After drying over anhydrous sodium sulfate, the solvent was evaporated and the oily product was purified on silica gel column eluting with 15% ethyl acetate-hexanes containing 0.25% triethylamine. Fractions were checked on silica gel TLC plate and product-containing fractions were combined. The solvent was evaporated under reduced pressure gave 3.4 parts title compound (62). The structure of the product was confirmed on the basis of ¹H NMR. The reaction proceeded as follows: 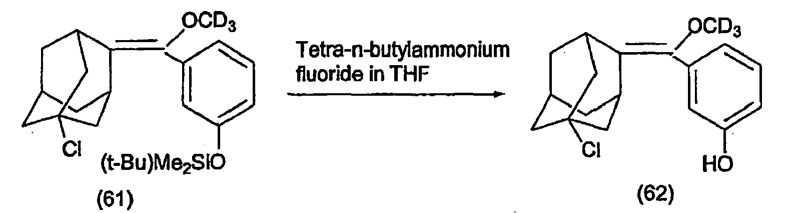
(c). Synthesis of [(3-phosphoryloxyphenyl) methoxy (D₃)) methylene]-5-chloroadamantane, disodium salt (63).
   Into a 500mL three-neck round bottom flask equippd with magenetic stirrer was added dry THF (75mL) under nitrogen and then 3.15mL of phosphorous oxychloride was added dropwise over 25 minutes (reaction flask was cooled in ice-water bath). A solution of 5 parts of alkene (62) in THF (75mL) containing 9.3 parts of anhydrous pyridine was added to the reaction flask over a period of 90 minutes. The reaction mixture was stirred at room temperature for 3 hours. TLC on silica gel plate showed the formation of new product. The solvent was evaporated to dryness and fresh THF (150ml) was added to the reaction flask. A solution containing 5.9 parts of 3-hydroxypropionitrile and 6.7 parts of anhydrous pyridine in dry THF (75mL) was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1 % triethylamine. Fractions were checked by TLC on silica gel plates and the product containing fractions were combined. The solvent was evaporated under reduced pressure gave 4.1 parts phosphate ester. The structure was confirmed on the basis of ¹H NMR. A solution of 0.85 parts of sodium hydroxide in water (4mL) was added dropwise to a solution of phosphate ester in THF (40mL) over 20 minutes and stirred for 2 hours. The reaction mixture was diluted with acetonitrile (15mL). The solid was filtered and washed with acetonitrile. The solid material was crystallized with a methanol and acetone mixture. The solid was filtered, washed with acetone and dried gave 3.4 parts of the title compound (63). The structure was confirmed on the basis of ¹H NMR. The reaction proceeded as follows: 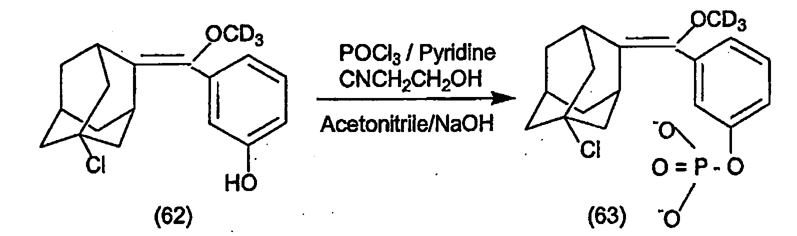
(d). Photooxidation of [(3-phosphoryloxyphenyl) methoxy (D₃)) methylene]-5-chloroadamantane, disodium salt (63).
   Alkene (63) was photooxidised as reported above, to give [(4-Methoxy (D3)) - 4-(3-phosphoryloxyphenyl)] spiro [1,2-dioxetane-3, 2'-5-chloroadamantane], disodium salt (64):

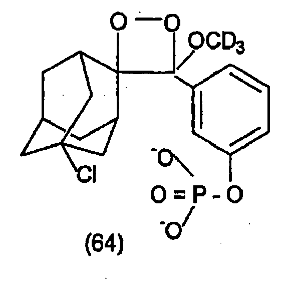

### Example X

This example illustrates the preparation of [(4-Methoxy-4-(3-phosphoryloxy-5-methoxy(D₃)phenyl)] spiro [1,2-dioxetane-3,2'-adamantane], disodium salt (72). The sequence of the reaction in accordance herewith:
(a). Synthesis of Methyl 3,5-dihydroxybenzoate (66), (a starting ester).
   In a 1000mL round bottom flask, 91.0g of 3,5-dihydroxybenzoic acid (65), 400mL of methanol and 15mL of concentrated sulfuric acid were heated at reflux for 23 hours. TLC on silica gel plates showed formation of the product. The solvent was evaporated under reduced pressure and the residue was stirred with deionized water (500ml) for 30 minutes. The solid was filtered off and air-dried for 24 hours yielding 81 parts of a crude product. A 31 parts of the crude product was purified by chromatography on silica gel column eluting with a 35% ethyl acetate-hexanes solution. Product-containing fractions were checked by TLC on silica gel plates and combined. The solvent was evaporated under reduced pressure giving 30.3 parts of the titile compound (66) as an off-white solid. The structure was confirmed by ¹H NMR. The reaction proceeded as follows : 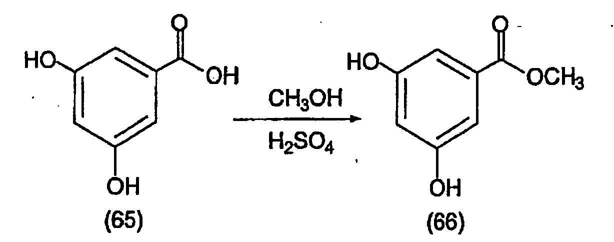
(b). Synthesis of Methyl 3-hydroxy-5-methoxy (D₃) benzoate (67), (a starting intermediate ester).
   In 1000mL three-neck flask with magnetic stirrer, 5.8 parts of sodium hydride, as a 60% dispersion in mineral oil was mixed with hexanes (100mL) under nitrogen atmosphere and stirred for 5 minutes. Stirring stopped for 10 minutes and the hexanes decanted off. This process was repeated with fresh hexanes (2x100mL) and suspended in anhydrous THF (150mL). A solution of 20.1 parts of methyl 3,5-dihydroxybenzoate (65) in THF (200mL) was added over 55 minutes and stirred for 1 hour at room temperature. The mixture was cooled to ∼8-10° C, a solution of 25 parts of iodomethane (D₃) In THF (75mL) was added over 45 minutes and slowly warmed to room temperature over 16 hours with stirring. The mixture was heated at 40-42° C for 72 hours with efficient condenser. The solution was concentrated to dryness under reduced pressure and dissolved in dichloromethane (300ml). The organic layer was washed with deionized water (2x250mL) and dried over anhydrous sodium sulfate. The organic solution was evaporated under resuced pressure and the crude product mixture was purified by chromatography on silica gel column eluting 5-15% ethyl acetate-hexanes. The fractions were checked by TLC on silica gel plates and product containing fractions were combined. The solvent was evaporated under reduced pressure gave 6.05 parts of title compound (67) as an off-white solid. The structure was confirmed on basis of ¹H NMR. The reaction proceeded as follows: 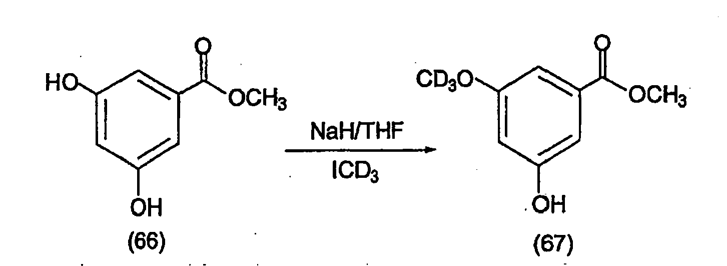
(c). Synthesis of Methyl 3-tert-butyldimethylsilyloxy-5-methoxy (D₃) benzoate (68), (a coupling ester).
   In to a 250ml round bottom flask, dry dimethylformamide (30mL), 5.95 parts of the deuteromethyl ester (67) and 6.36 parts of tert-butyldimethylsilyl chloride were added with stirring under nitrogen atmosphere. Six parts of imidazole was added in portions and stirring was continued for 2 hours. The reaction mixture was extracted with hexanes (3 x 150mL) and the hexane layer was washed with deionized water (2x250mL). The organic layer was dried over anhydrous sodium sulfate and the solvent was evaporated under reduced pressure to give 9.6 parts of the title compound (67) as oil. This material was pure by TLC on silica gel plates and the structure was confirmed by ¹H NMR. The material was prepared according to the following equation. 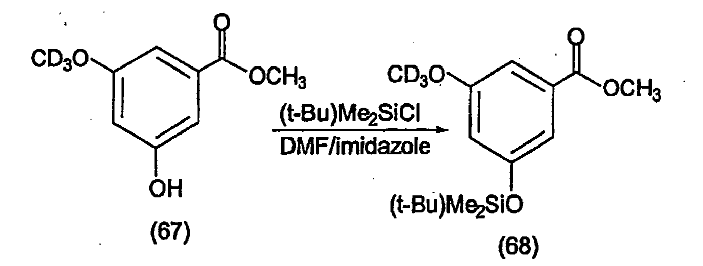
(d). Synthesis of [(3-t-butyldimethylsilyloxy-5-methoxy (D₃) phenyl) (2-methoxy)methylene]-adamantane (69), (a coupled intermediate ester).
   Into a 3000mL three-neck flask equipped with mechanical stirrer, pressure-equalizing addition funnel and nitrogen line was charged with anhydrous THF (250mL). Fifty-five parts of titanium tetrachloride was added dropwise over a period of 10 minutes. The suspension was stirred for 10 minutes and 47.3 parts of zinc was added in small portions over 15 minutes. The reaction mixture was heated under reflux for 4.5 hours and 110mL of triethylamine was added dropwise over 10 minutes. After refluxing one hour, a solution of 9.6 parts methyl 3-tert-butyldimethylsilyloxy-5-methoxy (D₃) benzoate (68) and 5.19 parts of 2-adamantanone (22) in anhydrous THF (125mL) was added over a period of 90 minutes and the reaction mixture was heated for one hour. The mixture on TLC silica gel plate showed the presence of the starting ester. An additional 1.03 parts of 2-adamantanone (22) in 30mL of dry THF was added dropwise over 30 minutes and refluxed for 16 hours. The mixture was cooled to room temperature, diluted with 500 mL of hexane and decanted. The residue was washed with hexanes (3x200mL). The combined hexanes layer was filtered and evaporated under reduced pressure to give an oily material which was purified by chromatography on silica gel column eluting with 2.5% ethyl acetate-hexanes with 0.25% triethylamine. Fractions were checked by TLC on silica gel plate and product containing fractions were combined. The solvent was evaporated under reduced pressure gave 11.63 parts of title compound(69) as oil. The structure was confirmed by ¹H NMR. The reaction proceeded as follows: 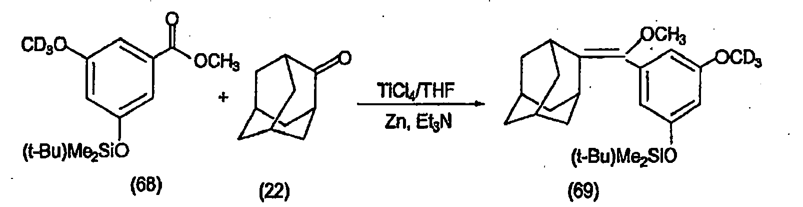
(e). Synthesis of [(3-Hydroxy-5-methoxy (D₃) phenyl) (2-methoxy) methylene]-adamantane (70), (alkene).
   To a solution 10.5 parts of purified silylalkene (69) in THF (125mL) was added 8.7 parts of 75 Wt% tetra-n-butylammonium fluoride in THF (50mL) over a period of 10 minutes and stirring was continued for 1.5 hours. TLC on silica gel plate showed the formation of new product. Solvent was evaporated under reduced pressure and the oily material was dissolved in dichloromethane (200mL) and washed with deionized water (2x100mL). After drying over anhydrous sodium sulfate, the solvent was evaporated and the oily product was purified on silica gel column eluting with 15-25% ethyl acetate-hexanes containing 0.25% triethylamine. Fractions were checked on silica gel TLC plate and product containing fractions were combined. The solvent was evaporated under reduced pressure gave 7 parts of title compound (70). The structure of the product was confirmed on the basis of ¹H NMR. The reaction proceeded as follows: 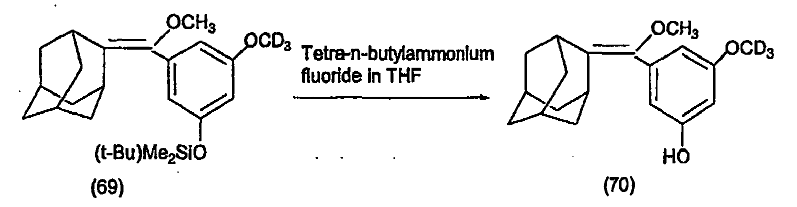
(f). Synthesis of [(3-phosphoryloxy-5-methoxy (D₃) phenyl) methoxy) methylene] adamantane, disodium salt (71).
   Into a 1000mL three-neck round bottom flask equippd with magenetic stirrer was added 100mL of dry THF under nitrogen and then 5.4 mL of phosphorous oxychloride was added dropwise over 25 minutes (reaction flask was cooled in ice-water bath). A solution of 5.82 parts of alkene (70) in 125mL of THF containing 16 parts of anhydrous pyridine was added to the reaction flask over a period of 90 minutes. The reaction mixture was stirred at room temperature for 3 hours. TLC on silica gel plate showed the formation of new product. The solvent was evaporated to dryness and 150mL of THF was added to the reaction flask. A solution containing 10.1 parts of 3-hydroxypropionitrile and 11.4 parts of anhydrous pyridine in 100mL of dry THF was added dropwise to the reaction mixture over 25 minutes and was stirred for 15 hours at room temperature. The reaction was cooled to ice-water temperature and the solid was filtered and washed with cold THF. The solvent was evaporated and oily material was chromatographed on silica gel column eluting with 75% ethyl acetate-hexane containing 0.1 % triethylamine. Fractions were checked by TLC on silica gel plate and the desired fractions were combined and evaporated under reduced pressure to gave 6.1 parts of phosphate ester as thick oil. The structure was confirmed on the basis of ¹H NMR. The phosphate ester was dissolved in 50 mL of dry THF and a solution of 1.3 parts of sodium hydroxide in 5 mL of water was added dropwise. Stirring was continued for two hours and the reaction mixture was diluted with 10 mL of acetonitrile. The solid was filtered and washed with acetonitrile. The solid material was crystallized from a methanol and acetone mixture. The solid was filtered, washed with acetone and then dried, to yield 4.8 parts of compound (71). The structure was confirmed on the basis of ¹H NMR. The reaction proceeded as follows: 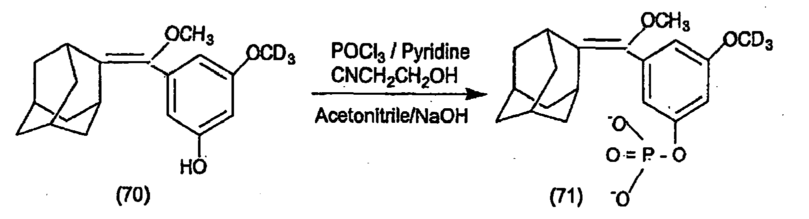
(g). Photooxidation of [(3-phosphoryloxy-5-methoxy (D₃) phenyl) methoxy) methylene] adamantane, disodium salt (71).
   Alkene (71) was photooxldised as reported above, to give [(4-Methoxy-4-(3-phosphoryloxy-5-methoxy(D₃)phenyl)] spiro [1,2-dioxetane-3,2'-adamantane], disodium salt (72):

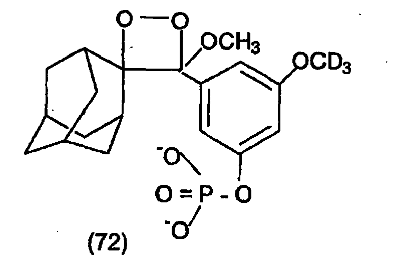

Having, thus, described the invention, what is claimed is:

## Claims

1. A 1, 2-dioxetane corresponding to the formula 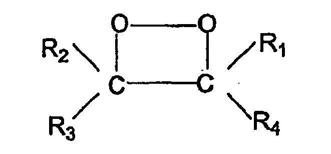 wherein R₁, R₂, R₃ and R₄ are each carbon containing organic groups in which at least one of the groups has at least one deuterium atom or a deuterium atom-containing group.

2. The 1, 2-dioxetane of claim 1 which corresponds to the formula: 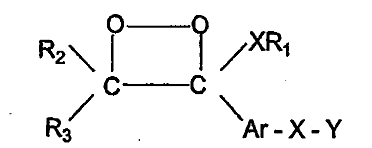 wherein when R₂ and R₃ form either (I) 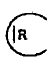 which is a cyclic, polycyclic or spiro-fused ring containing at least one carbon-carbon double bond or cabon-carbon triple bond in the ring or side chain, with or without heteroatoms, or (II) 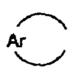 which is a cyclic, polycyclic or a spiro-fused ring containing a substituted or unsubstituted fused aromatic ring or a substituted or unsubstituted aromatic ring attached by linker arms, or (III) 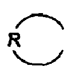 which is either a cyclic, substituted or unsubstituted polycyclic alkyl group which is spiro-fused to the dioxetane ring or (IV) R₂ and R₃ are each substituted or unsubstituted branched alkyl groups or cycloalkyl groups having 3 to 8 carbon atoms and being substituted in the ring or side chain, Ar is an aryl group which may be phenyl, substituted phenyl, naphthyl, substituted naphthyl, anthryl, substituted anthryl with or without a fluorescent group; Y is either hydrogen, alkyl, acetate, t-butyldimethylsilyl or other protecting groups, an enzyme cleavable group, or an antibody cleavable group; and R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, tinker-antigen, linker-biotin, inker-avidin, linker-protein, linker- carbohydrate or linker- lipid.

3. The 1, 2-dioxetane of claim 1 which corresponds to the formula: 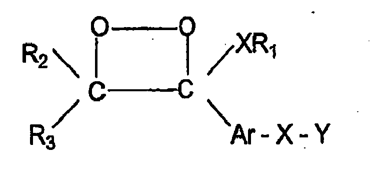 wherein R₂ and R₃ contain an isotopic hydrogen and either form (I) 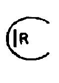 cyclic, polycyclic or a spiro-fused ring comprised of at least one carbon-carbon double bond or cabon-carbon triple bond in the ring or side chain with or without heteroatoms, (II) 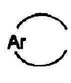 a cyclic, polycyclic or spiro-fused ring which is a substituted or unsubstituted fused aromatic ring or substituted or unsubstituted aromatic ring attached by linker arms, or (III) 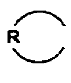 a substituted or unsubstituted cyclic, or polycyclic alkyl group which is spiro- fused to the dioxetane ring or (IV) R₂ and R₃ each contain 3 to 8 carbon atoms and are substituted or substituted branched alkyl or cycloalkyl groups having substitution in the ring or side chain, Ar, X and Y are as above; and R₁ is an organic group which is either cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-blotin, linker-avidin, linker protein, linker- carbohydrate or linker- lipid

4. The 1, 2-dioxetane of claim 1 which corresponds to the formula: 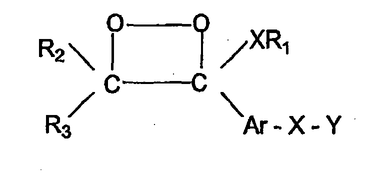 wherein R₂ and R₃ form either (I) 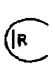 which is a cyclic, polycyclic or spiro-fused ring containing at least one carbon-carbon double bond or cabon-carbon triple bond in the ring or side chain, with or without heteroatoms, or (II) 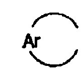 which is a cyclic, polycylic or a spiro-fused ring containing a substituted or unsubstituted fused aromatic ring or a substituted or unsubstituted aromatic ring attached by linker arms, or (III) 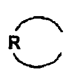 which is either a cyclic, a substituted or unsubstituted polycyclic alkyl group which is spiro-fused to the dioxetane ring or (IV) R₂ and R₃ are each 3 to 8 carbon atoms are substituted or substituted branched alkyl or cycloalkyl groups having substitution in the ring or side chain, Ar is an aryl group containing an isotopic hydrogen or isotopic hydrogen containing group, which may be phenyl, substituted phenyl, naphthyl, substituted naphthyl, anthtyl, substituted anthryl or any other aromatic or nonaromatic fluorescent or nonfluorescent group; X and Y are as defined above, and R₁ is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker- protein, linker carbohydrate or linker- lipid.

5. A method for generating light, which comprises:
(a) providing a 1, 2-dioxetane of the formula of claim 1.
(b) decomposing the 1, 2-dioxetane with an activating agent to give the corresponding carbonyl compounds.

6. The method of claim 5 wherein the 1, 2-dioxetane corresponds to the formula: 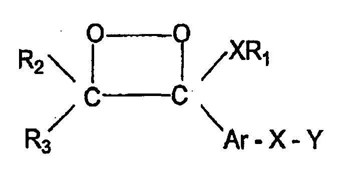 wherein at least one of R₁, R₂ or R₃ and Ar comprises a deuterium atom(s) or deuterium atom-containing group, X is sulfur or oxygen.

7. The method of claim 6 wherein: (a) when R₂ and R₃ form either (I) 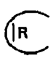 which is a cyclic, polycyclic or spiro-fused ring containing at least one carbon-carbon double bond or cabon-carbon triple bond in the ring or side chain, with or without heteroatoms, or (II) 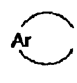 which is a cyclic, polycyclic or a spiro-fused ring containing a substituted or unsubstituted fused aromatic ring or a substituted or unsubstituted aromatic ring attached by linker arms, or (III) 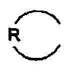 which is either a cyclic, substituted or unsubstituted polycyclic alkyl group which is spiro-fused to the dioxetane ring or (IV) R₂ and R₃ are each substituted or unsubstituted branched alkyl groups or cycloalkyl groups having 3 to 8 carbon atoms and being substituted in the ring or side chain, Ar is an aryl comprised which may be phenyl, substituted phenyl, naphthyl, substituted naphthyl, anthryl, substituted anthryl with or without a fluorescent group; Y is either hydrogen, alkyl, acetate, t-butyldimethylsilyl or other protecting groups, an enzyme cleavable group, or an antibody cleavable group; and R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, inker-avidin, linker-protein, linker- carbohydrate or linker- lipid.

8. The method of claim 6 wherein R₂ and R₃ contain an isotopic hydrogen and either form (I) 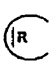 cyclic, polycyclic or a spiro-fused ring comprised of at least one carbon-carbon double bond or cabon-carbon triple bond in the ring or side chain with or without heteroatoms, (II) 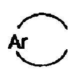 a cyclic, polycyclic or spiro-fused ring which is a substituted or unsubstituted fused aromatic ring or substituted or unsubstituted aromatic ring attached by linker arms, or (III) 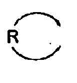 a substituted or unsubstituted cyclic, or polycyclic alkyl group which is spiro-fused to the dioxetane ring or (IV) R₂ and R₃ each contain 3 to 8 carbon atoms and are substituted or substituted branched alkyl or cycloalkyl groups having substitution in the ring or side chain, Ar, X and Y are as above; and R₁ is an organic group which is either cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker protein, linker- carbohydrate or linker- lipid

9. The method of claim 6 wherein R₂ and R₃ form either (I) 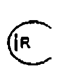 which is a cyclic, polycyclic or spiro-fused ring containing at least one carbon-carbon double bond or cabon-carbon triple bond in the ring or side chain, with or without heteroatoms, or (II) 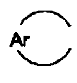 which is a cyclic, polycyclic or a spiro-fused ring containing a substituted or unsubstituted fused aromatic ring or a substituted or unsubstituted aromatic ring attached by linker arms, or (III) 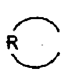 which is either a cyclic, a substituted or unsubstituted polycyclic alkyl group which is spiro-fused to the dioxetane ring or (IV) R₂ and R₃ are each 3 to 8 carbon atoms are substituted or substituted branched alkyl or cycloalkyl groups having substitution in the ring or side chain, Ar is an aryl compound containing an isotopic hydrogen or isotopic hydrogen containing group, which may be phenyl, substituted phenyl, naphthyl, substituted naphthyl, anthryl, substituted anthryl or any other aromatic or nonaromatic fluorescent or nonfluorescent group; X and Y are as defined above, and R₁ is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-blotin, linker-evidin, linker-protein, linker- carbohydrate or linker- lipid.

10. The method of claim 5 wherein the 1, 2-dioxetane is of the formula: 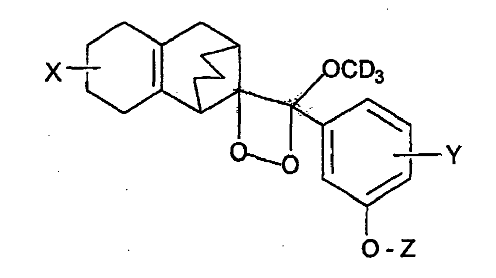 Wherein Z is a hydrogen, alkyl, acetate, t-butyldimethylsilyl or other protecting group, an enzyme cleaveable group or an antibody cleaveable group, Y is substitution in benzene ring such as hydrogen, a deuterium, a deuterium atom- containing group, halogen, hydroxy or substituted hydroxy, nitrile, alkyl, alkaryl, aralkyl, amino or substituted amino, nitro, aldehyde, acid, amide, aryl or substituted aryl and not limited to: X is substitution in polycyclic ring such as hydrogen, a deuterium, a deuterium atom-containing group, halogen, hydroxy or substituted hydroxy, nitrile, alkyl, alkaryl, aralkyl, amino or substituted amino, nitro, aldehyde, acid, amide, aryl or substituted aryl and not limited to.

11. The method of claim 5 wherein the 1, 2-dioxetane is of the formula: 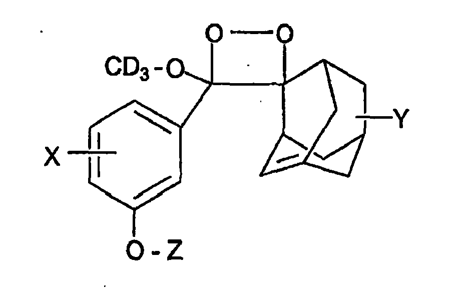 Wherein Z is a hydrogen, alkyl, acetate, t-butyldlmethylsilyl or other protecting group, an enzyme cleaveable group or an antibody cleaveable group, Y is substitution in benzene ring such as hydrogen, a deuterium, a deuterium atom- containing group, halogen, hydroxy or substituted hydroxy, nitrile, alkyl, alkaryl, aralkyl, amino or substituted amino, nitro, aldehyde, acid, amide, aryl or substituted aryl and not limited to: X is substitution in adamantene ring such as hydrogen, a deuterium, a deuterium atom-containing group, halogen, hydroxy or substituted hydroxy, nitrile, alkyl, alkaryl, aralkyl, amino or substituted amino, nitro, aldehyde, acid, amide, aryl or substituted aryl and not limited to.

12. The method of claim 5 wherein the 1, 2-dioxetane is of the formula: 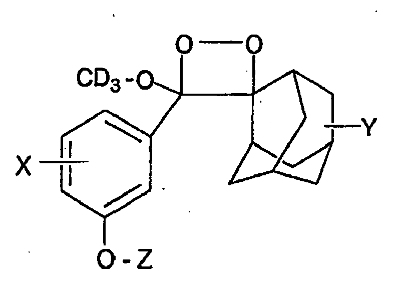 Wherein Z is selected from a group consisting of a hydrogen, alkyl, acetate, and t-butyldimethylsilyl protecting group, an enzyme cleaveable group or an antibody cleaveable group, Y is hydrogen, a deuterium atom, a deuterium atom-containing group, halogens, hydroxy or substituted hydroxy, nitrile, alkyl, alkaryl, aralkyl, amino or substituted amino, nitro, aldehyde, acid, amide, aryl or substituted aryl and not limited to; X hydrogen, a deuterium atom, a deuterium atom-containing group, halogen, hydroxy or substituted hydroxy, nitrile, alkyl, alkaryl, aralkyl, amino or substituted amino, nitro, aldehyde, acid, amide, aryl or substituted aryl and not limited to.

13. The method of claim 5 wherein the 1, 2-dioxetane is of the formula: 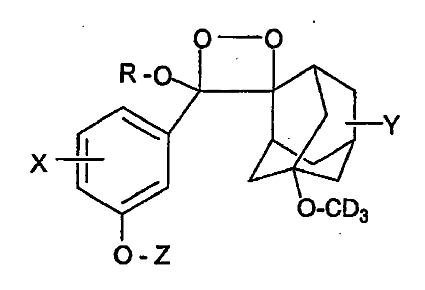 wherein R₁ is an organic group having an isotopic hydrogen and is selected from the group consisting of cyclic, linear or branched, halogenated or non-halogenated alkyl, aryl aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker antigen, linker-biotin, inker-avidin, linker-protein, linker- carbohydrate or linker- lipid; Z is hydrogen, alkyl, acetate and t-butyldimethylsilyl protecting group, an enzyme cleaveable group or an antibody cleaveable group, Y is hydrogen, a deuterium atom, a deuterium-containing group, halogens, hydroxy or substituted hydroxy, nitrile, alkyl, alkaryl, aralkyl, amino or substituted amino, nitro, aldehyde, acid, amide, aryl or substituted aryl and not limited to: X is substitution in adamantane ring such as hydrogen, a deuterium, a deuterium atom-containing group, halogens, hydroxy or substituted hydroxy, nitrile, alkyl, alkaryl, aralkyl, amino or substituted amino, nitro, aldehyde, acid, amide, aryl or substituted aryl and not limited to.

14. The method of claim 5 wherein the 1, 2-dioxetane is of the formula: 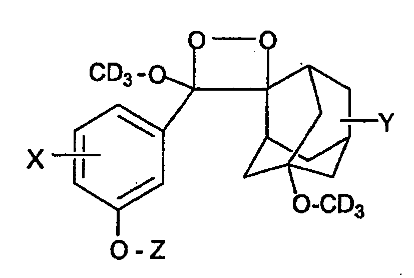 wherein Z is a hydrogen, alkyl, acetate and t-butyldimethylsilyl protecting group, an enzyme cleaveable group or an antibody cleaveable group, Y is hydrogen, a deuterium atom, a deuterium- containing group, halogen, hydroxy or substituted hydroxy, nitrile, alkyl, alkaryl, aralkyl, amino or substituted amino, nitro, aldehyde, acid, amide, aryl or substituted aryl and not limited to: X is hydrogen, a deuterium atom, a deuterium atom-containing group, halogen, hydroxy or substituted hydroxy, nitrile, alkyl, alkaryl, aralkyl, amino or substituted amino, nitro, aldehyde, acid, amide, aryl or substituted aryl and not limited to.

15. The method of claim 5 wherein the 1,2-dioxetane is of the formula: 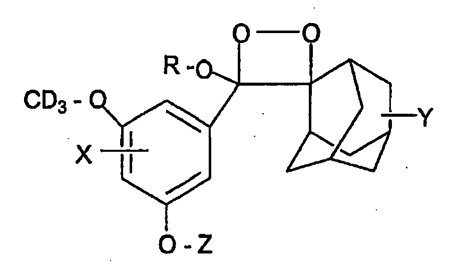 wherein R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, inker-avidin, linker- protein, linker- carbohydrate or linker- lipid; Z is a hydrogen, alkyl, acetate, t-butyldimethylsilyl protecting group, an enzyme cleaveable group or an antibody cleaveable group, Y is hydrogen, a deuterium atom, a deuterium-containing group, halogen, hydroxy or substituted hydroxy, nitrile, alkyl, alkaryl, aralkyl, amino or substituted amino, nitro, aldehyde, acid, amide, aryl or substituted aryl and not limited to: X is hydrogen, a deuterium atom, a deuterium atom-containing group, halogen, hydroxy or substituted hydroxy, nitrile, alkyl, alkaryl, aralkyl, amino or substituted amino, nitro, aldehyde, acid, amide, aryl or substituted aryl and not limited to.

16. The 1, 2-dioxetane of claim 1 which is any of:
(a) [(4 -Methoxy (D₃)-4-(3- phosphoryloxyphenyl)] spiro [1, 2-dioxetane-3, 1 3-tricyclo [7.3.1.0 ^{2,7}] trideo-2, 7-ene], disodium salt;
(b) [(4-methoxy (D₃))-4-(3-phosphoryloxy-4-chlorophenyl)] spiro [1,2-dioxetane-3, 2'-(5-chloroadamantane)], disodium salt;
(c) [(4 -Methoxy (D₃)-4-(3- phosphoryloxy-4-chlorophenyl)] spiro [1, 2-dioxetane-3, 2'-adamantan-4, 5-ene], disodium salt;
(d) [(4-methoxy (D3))-4-(3-phosphoryloxy-4-chlorophenyl)] spiro [1, 2-dioxetane-3, 2'-adamantane], disodium salt;
(e) [(4-methoxy (D₃))4-(3-phosphoryloxyphenyl)] spiro [1, 2-dioxetane-3, 2'-(5-chloroadamantane)], disodium salt;
(f) [(4-methoxy (D₃))-4-(3-phosphoryloxyphenyl)] spiro [1, 2-dioxetane-3, 2'-adamantane], disodium salt;
(g) [(4-methoxy (D₃))-4-(3-phosphoryloxy-4-chlorophenyl)] spiro [1,2-dioxetane-3, 2'-(5-methoxyadamantane)], disodium salt;
(h) [(4-methoxy-4-(3-phosphoryloxy-4-chlorophenyl)] spiro [1, 2-dioxetane-3, 2'-(5-methoxy(D₃)adamantane)], disodium salt;
(i) [(4-methoxy (D₃))-4-(3-phosphoryloxy-4-chlorophenyl)] spiro [1, 2-dioxetane-3, 2'-(5- methoxy (D₃) adamantane)], disodium salt; and
(j) [(4-methoxy-4-(3-phosphoryloxy-5-methoxy (D₃) phenyl)] spiro [1,2-dioxetane-3, 2'-adamantane], disodium salt.

17. An alkene intermediate for the formation of the stable 1, 2-dioxetane of claim 2, the alkene corresponding to the formula: 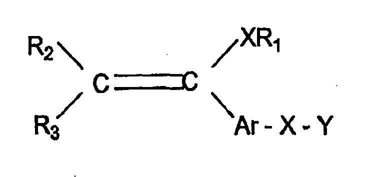 wherein at least one of R₁, R₂ or R₃ and Ar comprises a deuterium atom or deuterium atom-containing group, X is sulfur or oxygen and, wherein:
(a) when R₂ and R₃ form either (I) 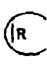 which is a cyclic, polycyclic or spiro-fused ring containing at least one carbon-carbon double bond or cabon-carbon triple bond in the ring or side chain, with or without heteroatoms, or (II) 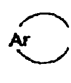 which is a cyclic, polycyclic or a spiro-fused ring containing a substituted or unsubstituted fused aromatic ring or a substituted or unsubstituted aromatic ring attached by linker arms, or (III) 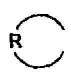 which is either a cyclic, substituted or unsubstituted polycyclic alkyl group which is spiro-fused to the dioxetane ring or (IV) R₂ and R₃ are each substituted or unsubstituted branched alkyl groups or cycloalkyl groups having 3 to 8 carbon atoms and being substituted in the ring or side chain, Ar is an aryl comprised which may be phenyl, substituted phenyl, naphthyl, substituted naphthyl, anthryl, substituted anthryl with or without a fluorescent group; Y is either hydrogen, alkyl, acetate, t-butyldimethylsilyl or other protecting groups, an enzyme cleavable group, or an antibody cleavable group; and R₁ is an organic group having an isotopic hydrogen( deuterium atom) and is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, inker-avidin, linker- protein, linker- carbohydrate or linker- lipid, or
(b) when R₂ and R₃ contain an isotopic hydrogen and either form (I) 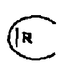 cyclic, polycyclic or a spiro-fused ring comprised of at least one carbon-carbon double bond or cabon-carbon triple bond in the ring or side chain with or without heteroatoms, (II) 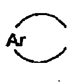 a cyclic, polycyclic or spiro-fused ring which is a substituted or unsubstituted fused aromatic ring or substituted or unsubstituted aromatic ring attached by linker arms, or (III) 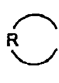 a substituted or unsubstituted cyclic, or polycyclic alkyl group which is spiro- fused to the dioxetane ring or (IV) R₂ and R₃ each contain 3 to 8 carbon atoms and are substituted or substituted branched alkyl or cycloalkyl groups having substitution in the ring or side chain, Ar, X and Y are as above; and R₁ is an organic group which is either cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnltrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker-protein, linker- carbohydrate or linker- lipid, or
(c) when R₂ and R₃ form either (I) 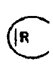 which is a cyclic, polycyclic or spiro-fused ring containing at least one carbon-carbon double bond or cabon-carbon triple bond in the ring or side chain, with or without heteroatoms, or (II) 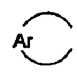 which is a cyclic, polycyclic or a spiro-fused ring containing a substituted or unsubstituted fused aromatic ring or a substituted or unsubstituted aromatic ring attached by linker arms, or (III) 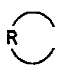 which is either a cyclic, a substituted or unsubstituted polycyclic alkyl group which is spiro-fused to the dioxetane ring or (IV) R₂ and R₃ are each 3 to 8 carbon atoms are substituted or substituted branched alkyl or cycloalkyl groups having substitution in the ring or side chain, Ar is an aryl compound containing an isotopic hydrogen or isotopic hydrogen containing group, which may be phenyl, substituted phenyl, naphthyl, substituted naphthyl, anthryl, substituted anthryl or any other aromatic or nonaromatic fluorescent or nonfluorescent group; X and Y are as defined above, and R₁ is selected from the group consisting of cyclic, linear or branched, halogenated or non-halogenated alkyl, aryl, aralkyl, alkaryl, heteroalkyl, heteroaryl, cycloalkyl, cycloheteroalkyl, alkyletheralkyl, alkyletheraryl, alkyl(etheralkyl)₂, alkyl(etheralkyl)₃, alkyletherhaloalkyl, alkyl(etherhaloalkyl)₂, alkylalkene, alkylalkyne, arylalkene, arylalkyne, alkylalcohol, alkylnitrile, alkylamine, alkylacid or the inorganic salts thereof, haloalkylalcohol, haloalkylnitrile, haloalkylamine, haloalkylacid or inorganic salts thereof, linker-flourescent molecule, linker-antibody, linker-antigen, linker-biotin, linker-avidin, linker- protein, linker- carbohydrate or linker- lipid.

18. The method of claim 5 wherein the activating agent is an enzyme from a biological source.
